# EUROPEAN PATENT APPLICATION

(11) **EP 4 394 786 A1**
(43) Date of publication of application: **03.07.2024**
(21) Application number: 22875765.4
(22) Date of filing: 08.09.2022
(51) Int. Cl.: G16H 20/10, C12M 1/00

(54) **PHARMACEUTICAL ASSISTANCE DEVICE, OPERATION METHOD FOR PHARMACEUTICAL ASSISTANCE DEVICE, AND OPERATION PROGRAM FOR PHARMACEUTICAL ASSISTANCE DEVICE**

(30) Priority: 29.09.2021 JP 2021160000
(71) Applicant: FUJIFILM Corporation, Tokyo 106-8620 (JP)
(72) Inventor: WANG, Caihua, Ashigarakami-gun, Kanagawa 258-8577 (JP)
(74) Representative: Hoffmann Eitle
(86) International application number: PCT/JP2022/033705
(87) International publication number: WO 2023/053893

(57) **Abstract**

Provided is a pharmaceutical support device including a processor. The processor is configured to: use a plurality of machine learning models that output prediction data indicating preservation stability of a candidate preservation solution, which is a candidate for a preservation solution for a biopharmaceutical, at a future time point and that are provided for a plurality of types of the preservation stability, respectively; perform a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and input the prediction data obtained in the prediction process in a previous stage to the machine learning model in the prediction process in a subsequent stage.

## Description

### BACKGROUND OF THE INVENTION

### 1. Field of the Invention

The technology of the present disclosure relates to a pharmaceutical support device, a method for operating a pharmaceutical support device, and a program for operating a pharmaceutical support device.

### 2. Description of the Related Art

In recent years, biopharmaceuticals have attracted attention due to high drug efficacy and low side effects. The biopharmaceutical has, for example, a protein, such as interferon or an antibody, as an active ingredient. The biopharmaceutical is preserved in a preservation solution. It is important to prescribe a preservation solution (also referred to as formulation prescription) suitable for the biopharmaceutical in order to stably maintain the quality of the biopharmaceutical.

The preservation solution includes a buffer solution, an additive, and a surfactant. The prescription of the preservation solution is, for example, the type and concentration of each of the buffer solution, the additive, and the surfactant and a hydrogen ion exponent (Potential of Hydrogen (pH) value) of the preservation solution.

In the related art, in a case where the prescription of the preservation solution is determined, a plurality of types of preservation solutions are prepared, for example, while changing a combination of the buffer solution, the additive, and the surfactant, and the preservation stability of a protein in each preservation solution is checked by an actual test. However, it takes a lot of time and effort to perform this operation. Therefore, for example, as in "Theresa K. Cloutier, etc., Machine Learning Models of Antibody-Excipient Preferential Interactions for Use in Computational Formulation Design, Mol. Pharmaceuticals, 17, 9, 3589-3599, 2020." (hereinafter, referred to as Document 1), a technique has been proposed that predicts preservation stability of a protein against an additive on the basis of information of the protein in a biopharmaceutical and information of the additive in a preservation solution, using a molecular dynamics (MD) method or a machine learning model, without performing any test.

### SUMMARY OF THE INVENTION

For the preservation stability of the preservation solution, there are various types of preservation stability, such as preservation stability of the protein against aggregation and preservation stability of the protein against temperature, including the preservation stability of the protein against the additive described in Document 1. In a case where a plurality of types of preservation stability are collectively predicted in a form of integrated preservation stability, prediction accuracy is reduced. As a result, there is a concern that it will not be possible to successfully search for the prescription of the preservation solution suitable for the biopharmaceutical. However, the plurality of types of preservation stability affect each other. Therefore, even in a case where the plurality of types of preservation stability are predicted individually, there is also a concern that it will not be possible to successfully search for the prescription of the preservation solution suitable for the biopharmaceutical.

An embodiment according to the technology of the present disclosure provides a pharmaceutical support device, a method for operating a pharmaceutical support device, and a program for operating a pharmaceutical support device that can reduce a concern that it will not be possible to successfully search for a prescription of a preservation solution suitable for a biopharmaceutical.

According to the present disclosure, there is provided a pharmaceutical support device comprising a processor. The processor is configured to: use a plurality of machine learning models that output prediction data indicating preservation stability of a candidate preservation solution, which is a candidate for a preservation solution for a biopharmaceutical, at a future time point and that are provided for a plurality of types of the preservation stability, respectively; perform a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and input the prediction data obtained in the prediction process in a previous stage to the machine learning model in the prediction process in a subsequent stage.

Preferably, the plurality of machine learning models provided for the plurality of types of preservation stability, respectively, are models corresponding to at least two of preservation stability of a protein included in the biopharmaceutical against aggregation, preservation stability of the protein against temperature, and preservation stability of the protein against temporal deterioration.

Preferably, the processor is configured to input prescription information of the candidate preservation solution actually prepared to the machine learning model.

Preferably, the machine learning model outputs a reliability degree of the prediction data together with the prediction data, and the processor is configured to input the reliability degree obtained in the prediction process in the previous stage to the machine learning model in the prediction process in the subsequent stage.

Preferably, the processor is configured to input a feature amount derived on the basis of protein information related to the protein included in the biopharmaceutical to the machine learning model.

Preferably, the feature amount includes at least one of a solvent accessible surface area of the protein, a spatial aggregation propensity of the protein, a space charge map of the protein, or an indicator showing compatibility between the protein and an additive included in the candidate preservation solution.

Preferably, the measurement data is time-series data measured at at least two time points.

Preferably, the prescription information related to the prescription of the candidate preservation solution to be predicted includes at least one of a type of each of a buffer solution, an additive, and a surfactant included in the candidate preservation solution, a concentration of each of the buffer solution, the additive, and the surfactant, or a hydrogen ion exponent of the candidate preservation solution.

Preferably, the measurement data includes at least one of aggregation analysis data of sub-visible particles of the protein in the candidate preservation solution included in the biopharmaceutical, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

Preferably, the protein included in the biopharmaceutical is an antibody.

According to the present disclosure, there is provided a method for operating a pharmaceutical support device. The method comprises: using a plurality of machine learning models that output prediction data indicating preservation stability of a candidate preservation solution, which is a candidate for a preservation solution for a biopharmaceutical, at a future time point and that are provided for a plurality of types of the preservation stability, respectively; performing a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and inputting the prediction data obtained in the prediction process in a previous stage to the machine learning model in the prediction process in a subsequent stage.

According to the present disclosure, there is provided a program for operating a pharmaceutical support device. The program causes a computer to execute a process comprising: using a plurality of machine learning models that output prediction data indicating preservation stability of a candidate preservation solution, which is a candidate for a preservation solution for a biopharmaceutical, at a future time point and that are provided for a plurality of types of the preservation stability, respectively; performing a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and inputting the prediction data obtained in the prediction process in a previous stage to the machine learning model in the prediction process in a subsequent stage.

According to the technology of the present disclosure, it is possible to provide a pharmaceutical support device, a method for operating a pharmaceutical support device, and a program for operating a pharmaceutical support device that can reduce a concern that it will not be possible to successfully search for a prescription of a preservation solution suitable for a biopharmaceutical.

### BRIEF DESCRIPTION OF THE DRAWINGS

Fig. 1 is a diagram illustrating a pharmaceutical support server and an operator terminal.
Fig. 2 is a diagram illustrating information exchanged between the pharmaceutical support server and the operator terminal in prediction of preservation stability of a protein against aggregation in a first stage.
Fig. 3 is a diagram illustrating information exchanged between the pharmaceutical support server and the operator terminal in prediction of preservation stability of the protein against temperature in a second stage.
Fig. 4 is a diagram illustrating information exchanged between the pharmaceutical support server and the operator terminal in prediction of preservation stability of the protein against temporal deterioration in a third stage.
Fig. 5 is a diagram illustrating first predicted prescription information.
Fig. 6 is a diagram illustrating second predicted prescription information.
Fig. 7 is a diagram illustrating third predicted prescription information.
Fig. 8 is a diagram illustrating first measured prescription information.
Fig. 9 is a diagram illustrating second measured prescription information.
Fig. 10 is a diagram illustrating third measured prescription information.
Fig. 11 is a diagram illustrating a series of flows of preparation of a prepared solution, addition of an antibody, a stress test, and measurement of measurement data and the measurement data.
Fig. 12 is a diagram illustrating first measurement data.
Fig. 13 is a diagram illustrating second measurement data.
Fig. 14 is a diagram illustrating third measurement data.
Fig. 15 is a block diagram illustrating a computer constituting the pharmaceutical support server.
Fig. 16 is a block diagram illustrating processing units of a CPU of the pharmaceutical support server.
Fig. 17 is a diagram illustrating a preservation stability prediction model.
Fig. 18 is a diagram illustrating an outline of prediction in the first stage using an aggregation preservation stability prediction model.
Fig. 19 is a diagram illustrating details of the prediction in the first stage using the aggregation preservation stability prediction model.
Fig. 20 is a table illustrating learning data for the aggregation preservation stability prediction model.
Fig. 21 is a diagram illustrating an outline of a process of the aggregation preservation stability prediction model in a learning phase.
Fig. 22 is a diagram illustrating an outline of the prediction in the second stage using a temperature preservation stability prediction model.
Fig. 23 is a diagram illustrating details of the prediction in the second stage using the temperature preservation stability prediction model.
Fig. 24 is a table illustrating learning data for the temperature preservation stability prediction model.
Fig. 25 is a diagram illustrating an outline of a process of the temperature preservation stability prediction model in the learning phase.
Fig. 26 is a diagram illustrating an outline of prediction in a third stage using the temporal deterioration preservation stability prediction model.
Fig. 27 is a diagram illustrating details of the prediction in the third stage using a temporal deterioration preservation stability prediction model.
Fig. 28 is a table illustrating learning data for the temporal deterioration preservation stability prediction model.
Fig. 29 is a diagram illustrating an outline of a process of the temporal deterioration preservation stability prediction model in the learning phase.
Fig. 30 is a flowchart illustrating a processing procedure of the pharmaceutical support server in the prediction in the first stage.
Fig. 31 is a flowchart illustrating a processing procedure of the pharmaceutical support server in the prediction in the second stage.
Fig. 32 is a flowchart illustrating a processing procedure of the pharmaceutical support server in the prediction in the third stage.
Fig. 33 is a diagram illustrating an outline of the prediction in the first stage, the prediction in the second stage, and the prediction in the third stage.
Fig. 34 is a diagram illustrating an aspect in which the aggregation preservation stability prediction model outputs a first reliability degree together with first prediction data.
Fig. 35 is a diagram illustrating an aspect in which the first reliability degree is also input to the temperature preservation stability prediction model and a second reliability degree is output from the temperature preservation stability prediction model together with second prediction data.
Fig. 36 is a diagram illustrating an aspect in which the first reliability degree and the second reliability degree are also input to the temporal deterioration preservation stability prediction model and a third reliability degree is output from the temporal deterioration preservation stability prediction model together with third prediction data.
Fig. 37 is a table illustrating learning data for the temperature preservation stability prediction model according to a second embodiment.
Fig. 38 is a diagram illustrating details of prediction in the first stage using the aggregation preservation stability prediction model according to the second embodiment.
Fig. 39 is a diagram illustrating a feature amount derivation unit that derives a feature amount on the basis of antibody information.
Fig. 40 is a diagram illustrating an aspect in which the feature amount is also input to the aggregation preservation stability prediction model.
Fig. 41 is a diagram illustrating an aspect in which the feature amount is also input to the temperature preservation stability prediction model.
Fig. 42 is a diagram illustrating an aspect in which the feature amount is also input to the temporal deterioration preservation stability prediction model.
Fig. 43 is a diagram illustrating an aspect in which measured prescription information of a prepared solution that has been actually prepared is input as predicted prescription information to the preservation stability prediction model.
Fig. 44 is a diagram illustrating another example of the preservation stability prediction model.
Fig. 45 is a diagram illustrating another example of the predicted prescription information.
Fig. 46 is a diagram illustrating another example of the measurement data.

### DESCRIPTION OF THE PREFERRED EMBODIMENTS

### [First Embodiment]

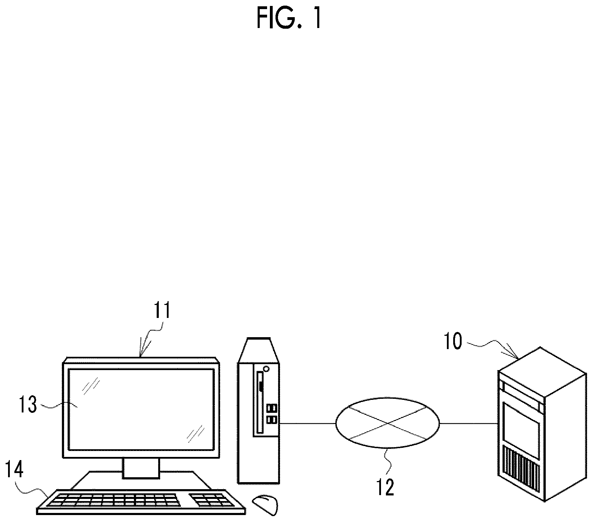

As illustrated in Fig. 1 as an example, a pharmaceutical support server 10 is connected to an operator terminal 11 through a network 12. The pharmaceutical support server 10 is an example of a "pharmaceutical support device" according to the technology of the present disclosure. The operator terminal 11 is installed, for example, in a pharmaceutical facility and is operated by an operator, such as a pharmacist, who is involved in the production of biopharmaceuticals in the pharmaceutical facility. The operator terminal 11 has a display 13 and an input device 14 such as a keyboard and a mouse. The network 12 is, for example, a wide area network (WAN) such as the Internet or a public communication network. In addition, in Fig. 1, only one operator terminal 11 is connected to the pharmaceutical support server 10. However, in practice, a plurality of operator terminals 11 in a plurality of pharmaceutical facilities are connected to the pharmaceutical support server 10.

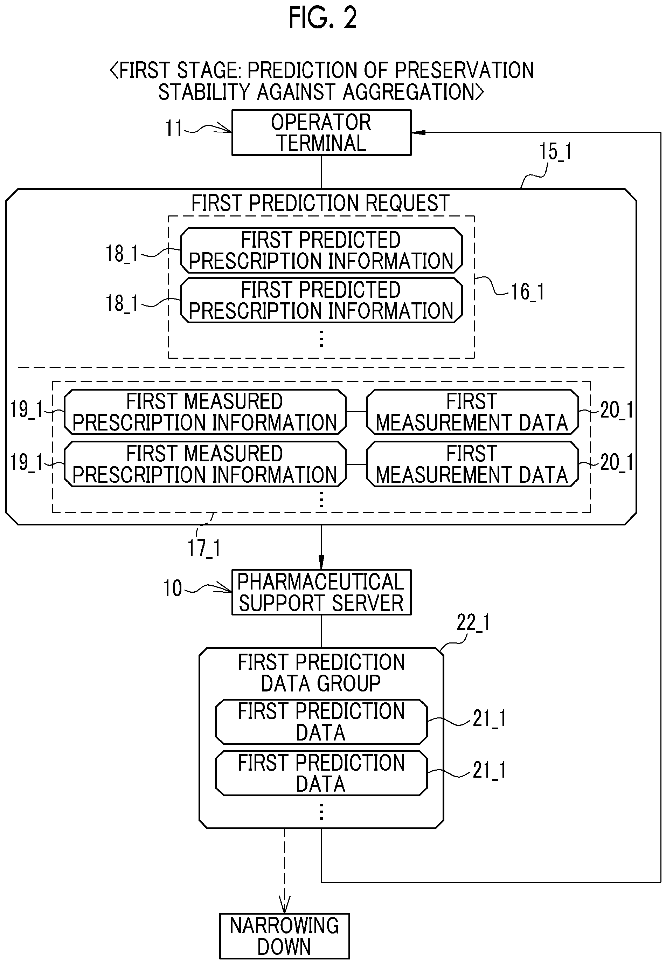

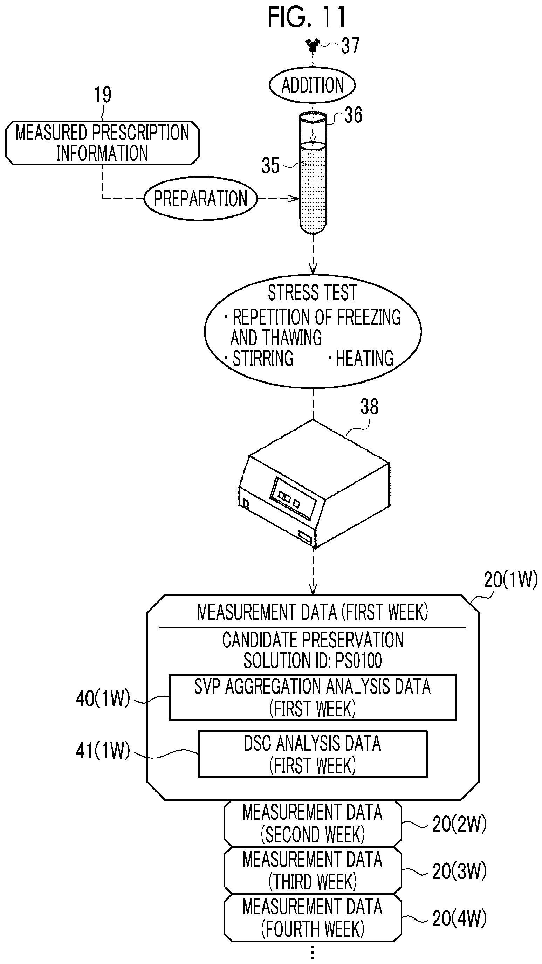

As illustrated in Fig. 2 as an example, the operator terminal 11 transmits a first prediction request 15_1 to the pharmaceutical support server 10. The first prediction request 15_1 is a request for the pharmaceutical support server 10 to predict preservation stability of a candidate preservation solution, which is a candidate for a preservation solution for a biopharmaceutical, in a first stage, more specifically, to predict preservation stability of an antibody 37 (see Fig. 11), which is an active ingredient of the biopharmaceutical, against aggregation. The first prediction request 15_1 includes a first predicted prescription information group 16_1 and a first measured prescription information and first measurement data set group 17_1.

The first predicted prescription information group 16_1 includes a plurality of first predicted prescription information items 18_1. The first predicted prescription information 18_1 is information related to the prescription of the candidate preservation solution (hereinafter, referred to as a target solution) to be predicted in the first stage. In this embodiment, the target solution is not actually prepared. The first measured prescription information and first measurement data set group 17_1 includes a plurality of first measured prescription information items 19_1 and a plurality of first measurement data items 20_1. The first measured prescription information 19_1 is information related to the prescription of a candidate preservation solution (hereinafter, referred to as a prepared solution) 35 that has been actually prepared (see Fig. 11). The first measurement data 20_1 is data obtained by actually measuring the preservation stability of the prepared solution 35 against aggregation. The operator operates the input device 14 to input the first predicted prescription information 18_1, the first measured prescription information 19_1, and the first measurement data 20_1. In addition, the first prediction request 15_1 also includes, for example, terminal identification data (ID) for uniquely identifying the operator terminal 11 which is a transmission source of the first prediction request 15_1, which is not illustrated.

In a case where the first prediction request 15_1 is received, the pharmaceutical support server 10 derives first prediction data 21_1 indicating the preservation stability of the target solution against aggregation at a future time point. The pharmaceutical support server 10 derives a plurality of first prediction data items 21_1 for a plurality of first predicted prescription information items 18_1, respectively. The pharmaceutical support server 10 delivers a first prediction data group 22_1 composed of the plurality of first prediction data items 21_1 to the operator terminal 11 which is the transmission source of the first prediction request 15_1.

In a case where the first prediction data group 22_1 is received, the operator terminal 11 displays the plurality of first prediction data items 21_1 on the display 13 such that the operator views the plurality of first prediction data items 21_1. The operator narrows the plurality of target solutions predicted in the first stage down to the target solutions to be predicted in the second stage on the basis of the plurality of first prediction data items 21_1.

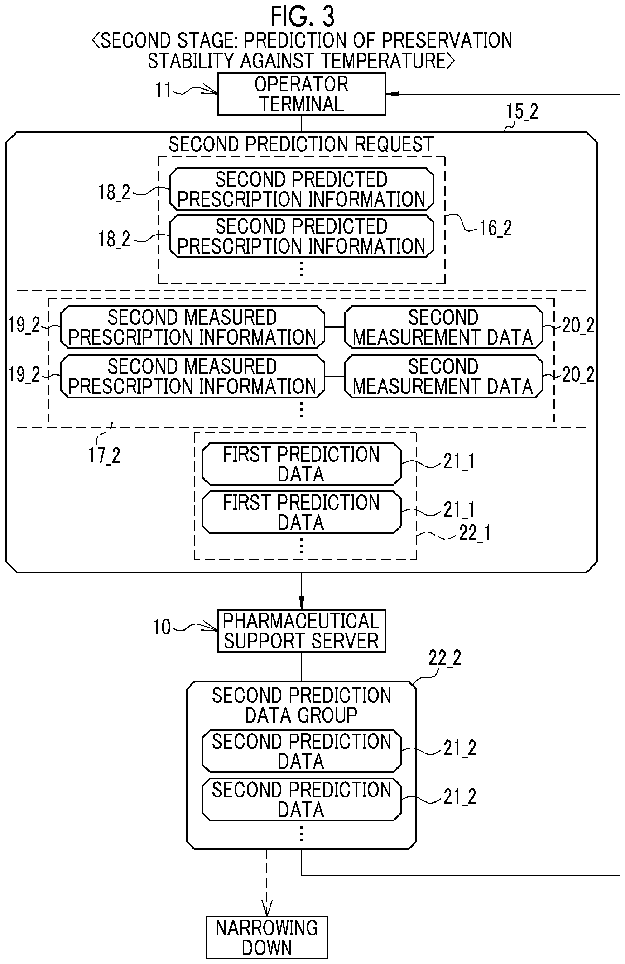

As illustrated in Fig. 3 as an example, after the prediction in the first stage is ended, the operator terminal 11 transmits a second prediction request 15_2 to the pharmaceutical support server 10. The second prediction request 15_2 is a request for the pharmaceutical support server 10 to predict the preservation stability of the target solutions, which have been narrowed down in the first stage, in the second stage, more specifically, to predict the preservation stability of the antibody 37 against temperature. The second prediction request 15_2 includes a second predicted prescription information group 16_2, a second measured prescription information and second measurement data set group 17_2, and the first prediction data group 22_1.

The second predicted prescription information group 16_2 includes a plurality of second predicted prescription information items 18_2. The second predicted prescription information 18_2 is information related to the prescription of the target solution narrowed down in the first stage. The second measured prescription information and second measurement data set group 17_2 includes a plurality of second measured prescription information items 19_2 and a plurality of second measurement data items 20_2. The second measured prescription information 19_2 is information related to the prescription of the prepared solution 35. The second measurement data 20_2 is data obtained by actually measuring the preservation stability of the prepared solution 35 against temperature. The operator also operates the input device 14 to input the second predicted prescription information 18_2, the second measured prescription information 19_2, and the second measurement data 20_2. The first prediction data group 22_1 delivered from the pharmaceutical support server 10 in the prediction in the first stage is attached without any change. In addition, the second prediction request 15_2 also includes, for example, the terminal ID of the operator terminal 11, which is not illustrated.

In a case where the second prediction request 15_2 is received, the pharmaceutical support server 10 derives second prediction data 21_2 indicating the preservation stability of the target solution against temperature at a future time point. The pharmaceutical support server 10 derives a plurality of second prediction data items 21_2 for a plurality of second predicted prescription information items 18_2, respectively. The pharmaceutical support server 10 delivers a second prediction data group 22_2 composed of the plurality of second prediction data items 21_2 to the operator terminal 11 which is the transmission source of the second prediction request 15_2.

In a case where the second prediction data group 22_2 is received, the operator terminal 11 displays the plurality of second prediction data items 21_2 on the display 13 such that the operator views the plurality of second prediction data items 21_2. The operator narrows the plurality of target solutions predicted in the second stage down to the target solutions to proceed to prediction in a third stage on the basis of the plurality of second prediction data items 21_2.

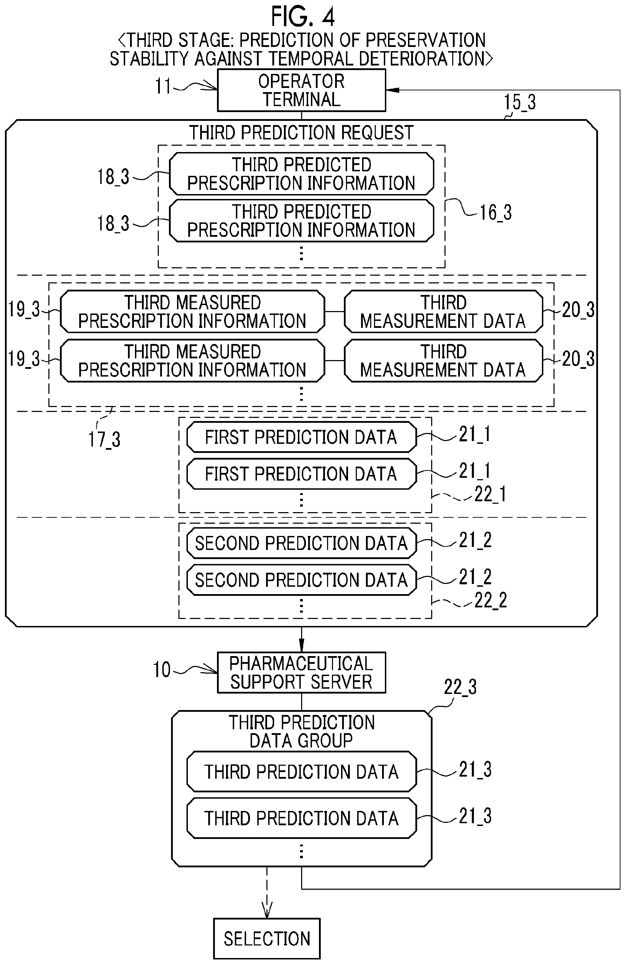

As illustrated in Fig. 4 as an example, after the prediction in the second stage is ended, the operator terminal 11 transmits a third prediction request 15_3 to the pharmaceutical support server 10. The third prediction request 15_3 is a request for the pharmaceutical support server 10 to predict the preservation stability of the target solutions, which have been narrowed down in the second stage, in the third stage, more specifically, to predict the preservation stability of the antibody 37 against temporal deterioration. The third prediction request 15_3 includes a third predicted prescription information group 16_3, a third measured prescription information and third measurement data set group 17_3, the first prediction data group 22_1, and the second prediction data group 22_2.

The third predicted prescription information group 16_3 includes a plurality of third predicted prescription information items 18_3. The third predicted prescription information 18_3 is information related to the prescriptions of the target solutions narrowed down in the second stage. The third measured prescription information and third measurement data set group 17_3 includes a plurality of third measured prescription information items 19_3 and a plurality of third measurement data items 20_3. The third measured prescription information 19_3 is information related to the prescription of the prepared solution 35. The third measurement data 20_3 is data obtained by actually measuring the preservation stability of the prepared solution 35 against temporal deterioration. The operator also operates the input device 14 to input the third predicted prescription information 18_3, the third measured prescription information 19_3, and the third measurement data 20_3. The first prediction data group 22_1 and the second prediction data group 22_2 delivered from the pharmaceutical support server 10 in the prediction in the first stage and the second stage are attached without any change. In addition, the third prediction request 15_3 also includes, for example, the terminal ID of the operator terminal 11, which is not illustrated.

In a case where the third prediction request 15_3 is received, the pharmaceutical support server 10 derives third prediction data 21_3 indicating preservation stability of the target solution against temporal deterioration at a future time point. The pharmaceutical support server 10 derives a plurality of third prediction data items 21_3 for a plurality of third predicted prescription information items 18_3, respectively. The pharmaceutical support server 10 delivers a third prediction data group 22_3 composed of the plurality of third prediction data items 21_3 to the operator terminal 11 which is the transmission source of the third prediction request 15_3.

In a case where the third prediction data group 22_3 is received, the operator terminal 11 displays the plurality of third prediction data items 21_3 on the display 13 such that the operator views the plurality of third prediction data items 21_3. The operator selects a target solution to be adopted as the preservation solution for the biopharmaceutical from the plurality of target solutions predicted in the second stage on the basis of the plurality of third prediction data items 21_3.

In addition, hereinafter, in a case where the first prediction request 15_1, the second prediction request 15_2, and the third prediction request 15_3 are not particularly distinguished from one another, they may be collectively referred to as a prediction request 15. Similarly, the first predicted prescription information group 16_1, the second predicted prescription information group 16_2, and the third predicted prescription information group 16_3 may be collectively referred to as a predicted prescription information group 16. In addition, the first measured prescription information and first measurement data set group 17_1, the second measured prescription information and second measurement data set group 17_2, and the third measured prescription information and third measurement data set group 17_3 may be collectively referred to as a measured prescription information and measurement data set group 17.

The first predicted prescription information 18_1, the second predicted prescription information 18_2, and the third predicted prescription information 18_3 may be collectively referred to as predicted prescription information 18. Further, the first measured prescription information 19_1, the second measured prescription information 19_2, and the third measured prescription information 19_3 may be collectively referred to as measured prescription information 19. In addition, the first measurement data 20_1, the second measurement data 20_2, and the third measurement data 20_3 may be collectively referred to as measurement data 20. Further, the first prediction data 21_1, the second prediction data 21_2, and the third prediction data 21_3 may be collectively referred to as prediction data 21. Furthermore, the first prediction data group 22_1, the second prediction data group 22_2, and the third prediction data group 22_3 may be collectively referred to as a prediction data group 22.

The predicted prescription information 18 is an example of "prescription information related to a prescription of a candidate preservation solution to be predicted" according to the technology of the present disclosure. The measured prescription information 19 is an example of "prescription information of a candidate preservation solution actually prepared" according to the technology of the present disclosure.

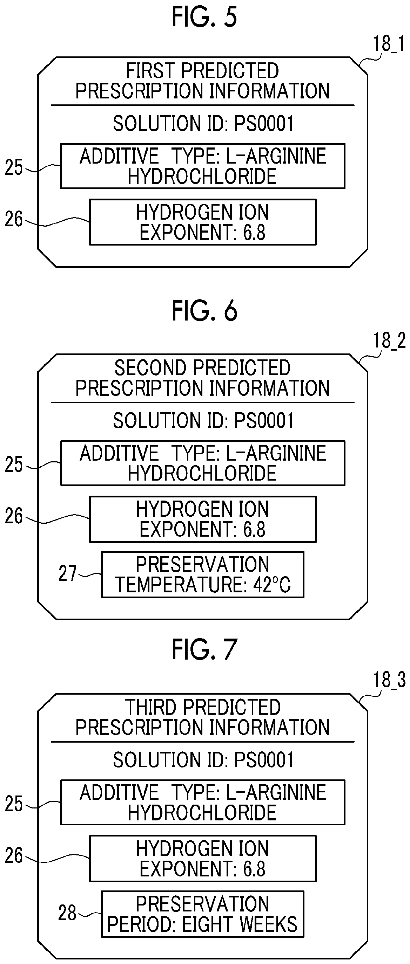

As illustrated in Fig. 5 as an example, the first predicted prescription information 18_1 includes a type 25 of an additive included in the target solution and a hydrogen ion exponent 26 of the target solution. As illustrated in Fig. 6 as an example, the second predicted prescription information 18_2 includes a preservation temperature 27 in addition to the type 25 of the additive and the hydrogen ion exponent 26. As illustrated in Fig. 7 as an example, the third predicted prescription information 18_3 includes a preservation period 28 in addition to the type 25 of the additive and the hydrogen ion exponent 26. A solution ID for uniquely identifying the target solution is given to the predicted prescription information 18. The operator creates the predicted prescription information 18, relying on the information of the antibody 37, his/her own experience, and the like. For example, the type 25 of the additive is digitized as follows: an additive A is digitized as "1"; and an additive B is digitized as "2".

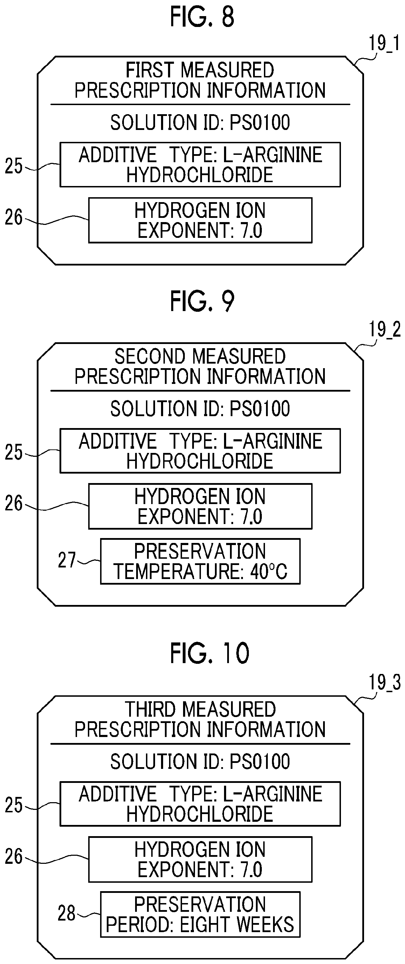

As illustrated in Fig. 8 as an example, the first measured prescription information 19_1 includes the type 25 of the additive included in the prepared solution 35 and the hydrogen ion exponent 26 of the prepared solution 35. As illustrated in Fig. 9 as an example, the second measured prescription information 19_2 includes the preservation temperature 27 in addition to the type 25 of the additive and the hydrogen ion exponent 26. As illustrated in Fig. 10 as an example, the third measured prescription information 19_3 includes the preservation period 28 in addition to the type 25 of the additive and the hydrogen ion exponent 26. The solution ID is also given to the measured prescription information 19. The measured prescription information 19 is created by using, for example, two types 25 of additives and dividing the hydrogen ion exponent 26 into, for example, 6.5, 7.0, and 7.5 at an interval of 0.5.

As illustrated in Fig. 11 as an example, the operator actually prepares the prepared solution 35 in a test tube 36 on the basis of the measured prescription information 19. Then, the antibody 37 is added to the prepared solution 35. Then, a stress test including the repetition of freezing and thawing, stirring, and heating is performed for a predetermined period (for example, 4 weeks to 8 weeks). During the stress test, the measurement data 20 is measured by a measurement device 38. Fig. 11 illustrates an example in which measurement data 20(1W), measurement data 20(2W), measurement data 20(3W), measurement data 20(4W), ... are measured in the first week, the second week, the third week, the fourth week, ... of the stress test, respectively. The measurement device 38 transmits the measurement data 20 to the operator terminal 11.

The measurement data 20 includes aggregation analysis data (hereinafter, referred to as SVP aggregation analysis data) 40 of sub-visible particles (SVPs) of the antibody 37 in the prepared solution 35 and analysis data (hereinafter, referred to as DSC analysis data) 41 of the antibody 37 in the prepared solution 35 by differential scanning calorimetry (DSC) as illustrated in the measurement data 20(1W) in the first week. The SVP aggregation analysis data 40 indicates the amount of SVPs of the antibody 37 in the prepared solution 35 that causes a reduction in the drug efficacy of the biopharmaceutical. It can be said that, as the value of the SVP aggregation analysis data 40 is larger, the preservation stability of the antibody 37 against aggregation is higher. The DSC analysis data 41 indicates values related to thermophysical property values such as the glass transition point and crystallization temperature of the antibody 37 in the prepared solution 35. It can be said that, as the value of the DSC analysis data 41 is smaller, the preservation stability of the antibody 37 against temperature is higher. The SVP aggregation analysis data 40 and the DSC analysis data 41 in each week are distinguished by attaching, for example, (1W) indicating the first week and (2W) indicating the second week.

In addition, in Fig. 11, for convenience, only one measurement device 38 is illustrated. However, in practice, various measurement devices 38 that measure the SVP aggregation analysis data 40 and the DSC analysis data 41 are prepared. Further, in Fig. 11, only the preparation of one prepared solution 35, the addition of the antibody 37, the stress test, and the measurement of the measurement data 20 are illustrated. However, in practice, the preparation, the addition, the stress test, and the measurement of the measurement data 20 are performed for each of a plurality of types of prepared solutions 35.

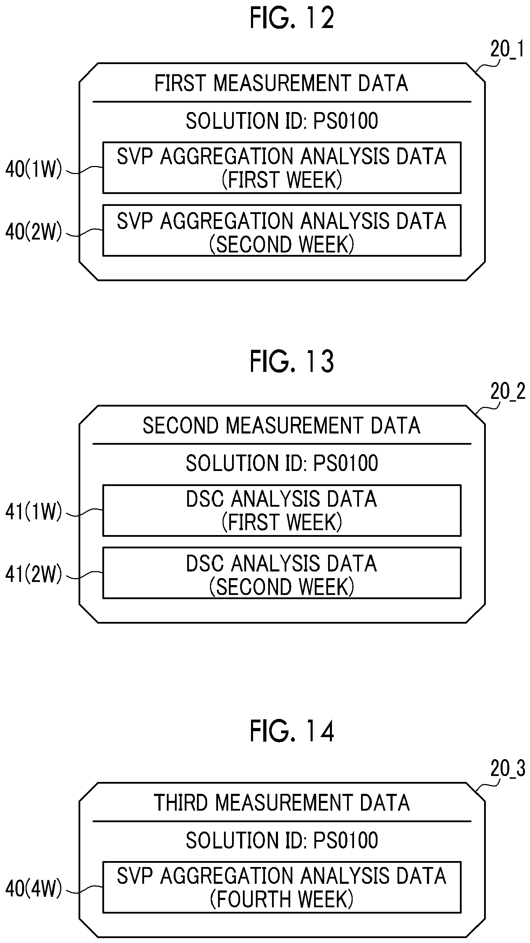

As illustrated in Fig. 12 as an example, the first measurement data 20_1 includes the SVP aggregation analysis data 40(1W) in the first week and the SVP aggregation analysis data 40(2W) in the second week. The SVP aggregation analysis data 40(1W) in the first week and the SVP aggregation analysis data 40(2W) in the second week are an example of "time-series data measured at at least two time points" according to the technology of the present disclosure. As illustrated in Fig. 13 as an example, the second measurement data 20_2 includes the DSC analysis data 41(1W) in the first week and the DSC analysis data 41(2W) in the second week. The DSC analysis data 41(1W) in the first week and the DSC analysis data 41(2W) in the second week are an example of the "time-series data measured at at least two time points" according to the technology of the present disclosure. As illustrated in Fig. 14 as an example, the third measurement data 20_3 includes the SVP aggregation analysis data 40(4W) in the fourth week. Similarly to the predicted prescription information 18 and the like, the measurement data 20 is given the solution ID for uniquely identifying the prepared solution.

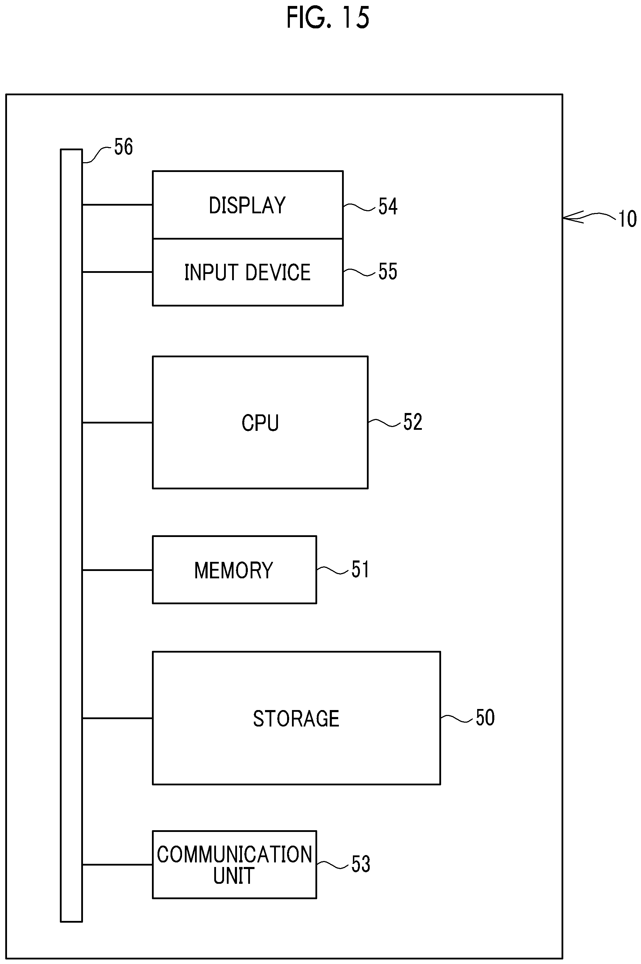

As illustrated in Fig. 15 as an example, a computer constituting the pharmaceutical support server 10 comprises a storage 50, a memory 51, a central processing unit (CPU) 52, a communication unit 53, a display 54, and an input device 55. These units are connected to one another through a bus line 56.

The storage 50 is a hard disk drive that is provided in the computer constituting the pharmaceutical support server 10 or that is connected to the computer through a cable or a network. Alternatively, the storage 50 is a disk array in which a plurality of hard disk drives are connected. The storage 50 stores, for example, a control program, such as an operating system, various application programs, and various types of data associated with these programs. In addition, a solid state drive may be used instead of the hard disk drive.

The memory 51 is a work memory used by the CPU 52 to perform processes. The CPU 52 loads the program stored in the storage 50 to the memory 51 and performs a process corresponding to the program. Therefore, the CPU 52 controls the overall operation of each unit of the computer. The CPU 52 is an example of a "processor" according to the technology of the present disclosure. In addition, the memory 51 may be provided in the CPU 52.

The communication unit 53 controls the transmission of various types of information to an external device such as the operator terminal 11. The display 54 displays various screens. The various screens have operation functions by a graphical user interface (GUI). The computer constituting the pharmaceutical support server 10 receives an input of an operation instruction from the input device 55 through the various screens. The input device 55 is, for example, a keyboard, a mouse, a touch panel, and a microphone for voice input.

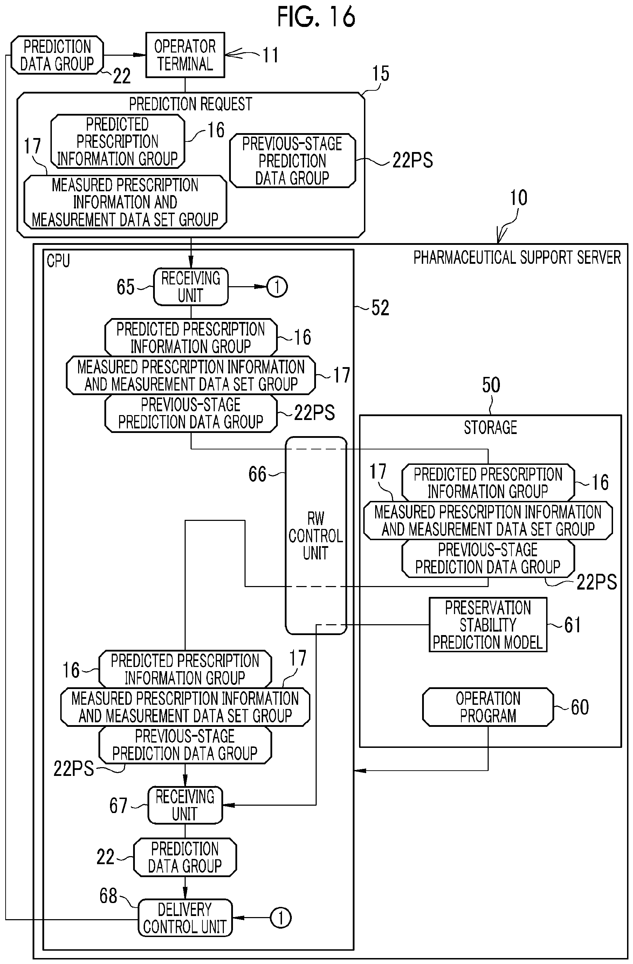

As illustrated in Fig. 16 as an example, an operation program 60 is stored in the storage 50 of the pharmaceutical support server 10. The operation program 60 is an application program for causing the computer to function as the pharmaceutical support server 10. That is, the operation program 60 is an example of "a program for operating a pharmaceutical support device" according to the technology of the present disclosure. A preservation stability prediction model 61 is also stored in the storage 50.

In a case where the operation program 60 is started, the CPU 52 of the computer constituting the pharmaceutical support server 10 functions as a receiving unit 65, a read and write (hereinafter, abbreviated to RW) control unit 66, a prediction unit 67, and a delivery control unit 68 in cooperation with the memory 51 and the like.

The receiving unit 65 receives the prediction request 15 from the operator terminal 11. As described above, the prediction request 15 includes the predicted prescription information group 16 and the measured prescription information and measurement data set group 17. In a case of the second prediction request 15_2 and the third prediction request 15_3, the first prediction data group 22_1 obtained in the prediction in the first stage and the second prediction data group 22_2 obtained in the prediction in the second stage (hereinafter, they are collectively referred to as a previous-stage prediction data group 22PS). Therefore, the receiving unit 65 receives the prediction request 15 to acquire the predicted prescription information group 16, the measured prescription information and measurement data set group 17, and the previous-stage prediction data group 22PS (in a case where the previous-stage prediction data group 22PS is included). The receiving unit 65 outputs the predicted prescription information group 16, the measured prescription information and measurement data set group 17, and the previous-stage prediction data group 22PS (in a case where the previous-stage prediction data group 22PS is included) to the RW control unit 66. In addition, the receiving unit 65 outputs the terminal ID of the operator terminal 11 (not illustrated) to the delivery control unit 68.

The RW control unit 66 controls the storage of various types of data in the storage 50 and the reading-out of various types of data in the storage 50. For example, the RW control unit 66 stores the predicted prescription information group 16, the measured prescription information and measurement data set group 17, and the previous-stage prediction data group 22PS (in a case where the previous-stage prediction data group 22PS is included) from the receiving unit 65 in the storage 50. In addition, the RW control unit 66 reads out the predicted prescription information group 16, the measured prescription information and measurement data set group 17, and the previous-stage prediction data group 22PS (in a case where the previous-stage prediction data group 22PS is included) from the storage 50 and outputs the predicted prescription information group 16, the measured prescription information and measurement data set group 17, and the previous-stage prediction data group 22PS (in a case where the previous-stage prediction data group 22PS is included) to the prediction unit 67. Furthermore, the RW control unit 66 reads out the preservation stability prediction model 61 from the storage 50 and outputs the preservation stability prediction model 61 to the prediction unit 67.

The prediction unit 67 derives the prediction data group 22 on the basis of the predicted prescription information group 16, the measured prescription information and measurement data set group 17, and the previous-stage prediction data group 22PS (in a case where the previous-stage prediction data group 22PS is included), using the preservation stability prediction model 61. The prediction unit 67 outputs the derived prediction data group 22 to the delivery control unit 68.

The delivery control unit 68 performs control to deliver the prediction data group 22 to the operator terminal 11 which is the transmission source of the prediction request 15. In this case, the delivery control unit 68 specifies the operator terminal 11, which is the transmission source of the prediction request 15, on the basis of the terminal ID from the receiving unit 65.

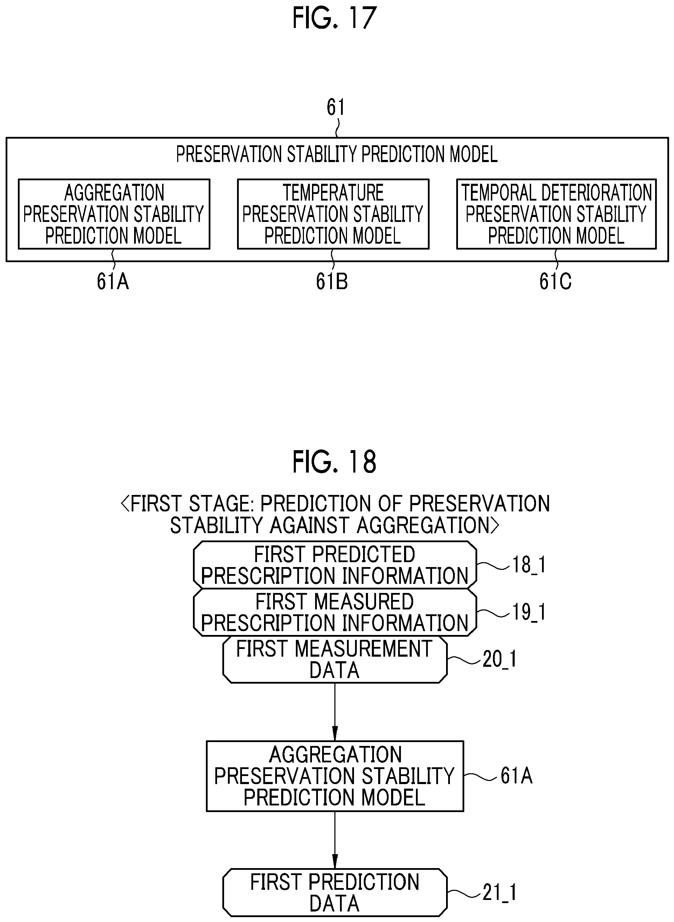

As illustrated in Fig. 17 as an example, the preservation stability prediction model 61 is composed of an aggregation preservation stability prediction model 61A, a temperature preservation stability prediction model 61B, and a temporal deterioration preservation stability prediction model 61C. The aggregation preservation stability prediction model 61A, the temperature preservation stability prediction model 61B, and the temporal deterioration preservation stability prediction model 61C are constructed by, for example, a neural network or a support vector regression (SVR) algorithm. The aggregation preservation stability prediction model 61A, the temperature preservation stability prediction model 61B, and the temporal deterioration preservation stability prediction model 61C are examples of a "machine learning model" according to the technology of the present disclosure.

As illustrated in Fig. 18 as an example, in the prediction in the first stage, the prediction unit 67 inputs the first predicted prescription information 18_1, the first measured prescription information 19_1, and the first measurement data 20_1 to the aggregation preservation stability prediction model 61A. Then, the first prediction data 21_1 is output from the aggregation preservation stability prediction model 61A. In addition, similarly to the first predicted prescription information 18_1 and the like, the first prediction data 21_1 is given the solution ID.

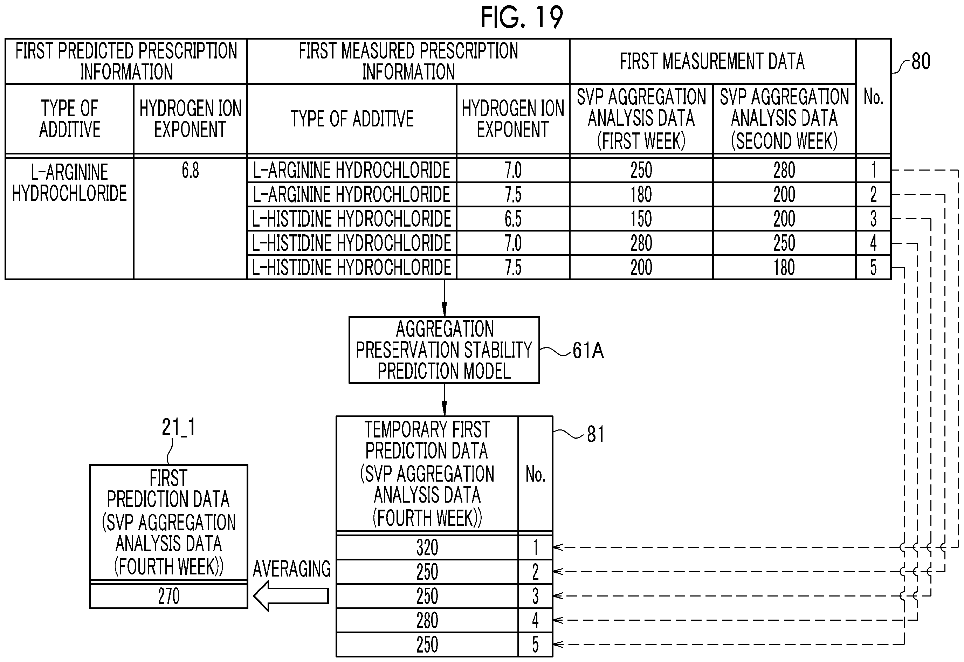

As illustrated in Tables 80 and 81 of Fig. 19 as an example, in the prediction in the first stage, the prediction unit 67 inputs the first predicted prescription information 18_1 and one of sets of the first measured prescription information 19_1 and the first measurement data 20_1 of a plurality of types of prepared solutions 35 to the aggregation preservation stability prediction model 61A such that temporary first prediction data is output from the aggregation preservation stability prediction model 61A. The prediction unit 67 repeats this process on the plurality of types of prepared solutions 35 such that a plurality of temporary first prediction data items are output from the aggregation preservation stability prediction model 61A. The prediction unit 67 calculates an average value of the plurality of temporary first prediction data items and sets the calculated average value as the first prediction data 21_1. The temporary first prediction data and thus the first prediction data 21_1 are data that predicts the SVP aggregation analysis data 40(4W) of the target solution in the fourth week which is represented by the first predicted prescription information 18_1. In addition, the first prediction data 21_1 may be the median value, maximum value, or minimum value of the plurality of temporary first prediction data items, instead of the average value, or may be a weighted average value calculated by multiplying the plurality of temporary first prediction data items by an appropriate weight.

Fig. 19 illustrates a case where five temporary first prediction data items of No. 1 to No. 5 are derived for five types of prepared solutions 35 of No. 1 to No. 5 in which the type 25 of the additive is any one of L-arginine hydrochloride or L-histidine hydrochloride and the hydrogen ion exponent is any one of 6.5, 7.0, or 7.5. For the temporary first prediction data, No. 1 is 320, No. 2, No. 3, and No. 5 are 250, and No. 4 is 280. Therefore, the first prediction data 21_1 is (320 + 250 + 250 + 280 + 250)/5 = 270.

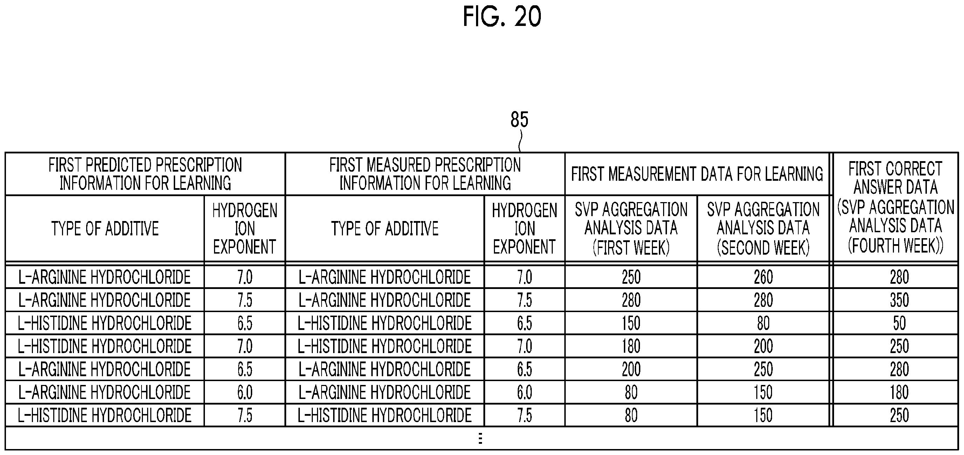

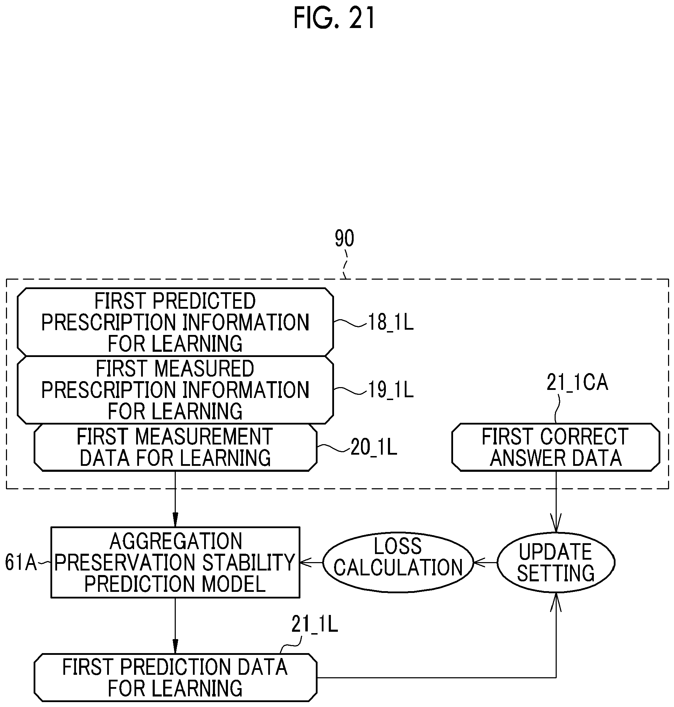

As in a table 85 illustrated in Fig. 20 as an example, as learning data 90 for the aggregation preservation stability prediction model 61A, a plurality of sets of first predicted prescription information 18_1L for learning, first measured prescription information 19_1L for learning, first measurement data 20_1L for learning, and first correct answer data 21_1CA (see Fig. 21) are stored in, for example, the storage 50. The same content is registered in the first predicted prescription information 18_1L for learning and the first measured prescription information 19_1L for learning. The first measurement data 20_1L for learning is data obtained by actually measuring the preservation stability of the prepared solution 35 in which the first measured prescription information 19_1L for learning has been registered and is the SVP aggregation analysis data 40(1W) in the first week and the SVP aggregation analysis data 40(2W) in the second week.

Similarly, the first correct answer data 21_1CA is data obtained by actually measuring the preservation stability of the prepared solution 35 in which the first measured prescription information 19_1L for learning has been registered. Specifically, the first correct answer data 21_1CA is the SVP aggregation analysis data 40(4W) in the fourth week. In addition, in a case where the first measurement data 20_1L for learning is not actually measured, a value prepared in advance or a mask indicating that there is no data is registered.

As illustrated in Fig. 21 as an example, the learning data 90 is given in a learning phase of the aggregation preservation stability prediction model 61A. The learning data 90 is a set of the first predicted prescription information 18_1L for learning, the first measured prescription information 19_1L for learning, the first measurement data 20_1L for learning, and the first correct answer data 21_1CA registered in the table 85 illustrated in Fig. 20. The first predicted prescription information 18_1L for learning, the first measured prescription information 19_1L for learning, and the first measurement data 20_1L for learning in the learning data 90 are input to the aggregation preservation stability prediction model 61A. As a result, first prediction data 21_1L for learning is output from the aggregation preservation stability prediction model 61A.

Loss calculation of the aggregation preservation stability prediction model 61A using a loss function is performed on the basis of the first prediction data 21_1L for learning and the first correct answer data 21_1CA. Then, the update setting of various coefficients of the aggregation preservation stability prediction model 61A is performed according to a result of the loss calculation, and the aggregation preservation stability prediction model 61A is updated according to the update setting.

In the learning phase of the aggregation preservation stability prediction model 61A, the series of processes of the input of the first predicted prescription information 18_1L for learning, the first measured prescription information 19_1L for learning, and the first measurement data 20_1L for learning to the aggregation preservation stability prediction model 61A, the output of the first prediction data 21_1L for learning from the aggregation preservation stability prediction model 61A, the loss calculation, the update setting, and the update of the aggregation preservation stability prediction model 61Ais repeated while the learning data 90 is exchanged. The repetition of the series of processes is ended in a case where the prediction accuracy of the first prediction data 21_1L for learning with respect to the first correct answer data 21_1CA has reached a predetermined set level. The aggregation preservation stability prediction model 61A in which the prediction accuracy has reached the set level in this manner is stored in the storage 50 and is used by the prediction unit 67. In addition, the learning may be ended in a case where the series of processes is repeated a set number of times, regardless of the prediction accuracy of the first prediction data 21_1L for learning with respect to the first correct answer data 21_1CA.

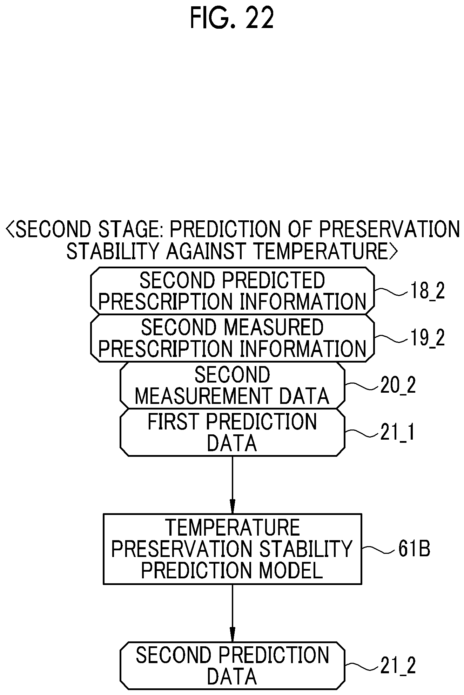

As illustrated in Fig. 22 as an example, in the prediction in the second stage, the prediction unit 67 inputs the second predicted prescription information 18_2, the preservation temperature 27 of the second measured prescription information 19_2, the second measurement data 20_2, and the first prediction data 21_1 to the temperature preservation stability prediction model 61B. Then, the second prediction data 21_2 is output from the temperature preservation stability prediction model 61B. In addition, similarly to the second predicted prescription information 18_2 and the like, the second prediction data 21_2 is also given the solution ID. The prediction in the second stage by the temperature preservation stability prediction model 61B is an example of a "prediction process in a subsequent stage" according to the technology of the present disclosure. In addition, the prediction in the first stage by the aggregation preservation stability prediction model 61A is an example of a "prediction process in a previous stage" according to the technology of the present disclosure, and the first prediction data 21_1 is an example of "prediction data obtained in the prediction process in the previous stage" according to the technology of the present disclosure.

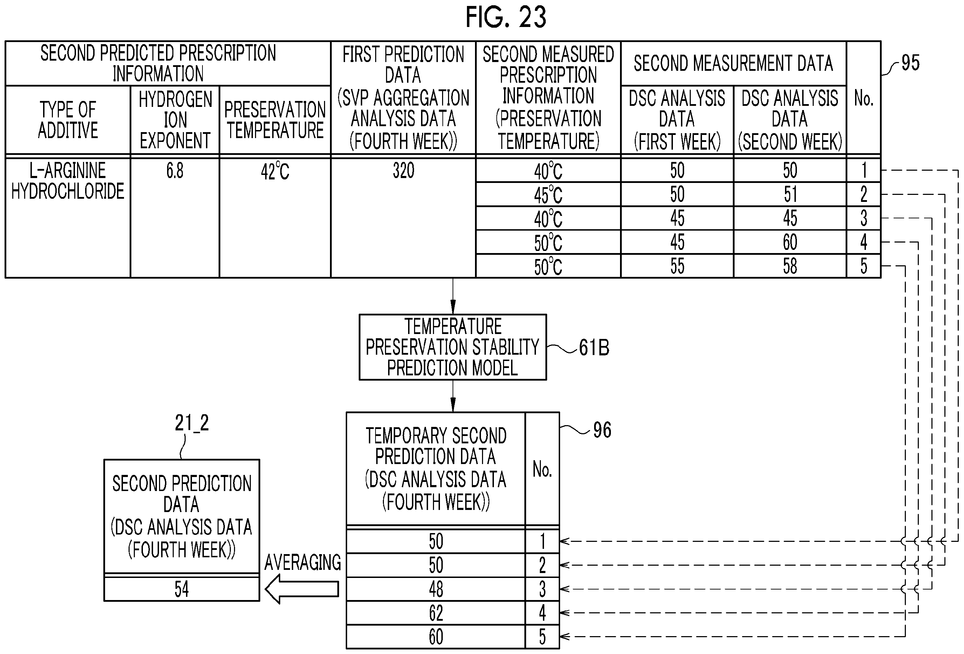

As illustrated in tables 95 and 96 of Fig. 23 as an example, in the prediction in the second stage, the prediction unit 67 inputs the second predicted prescription information 18_2, one of sets of the preservation temperature 27 of the second measured prescription information 19_2 and the second measurement data 20_2 of a plurality of types of prepared solutions 35, and the first prediction data 21_1 to the temperature preservation stability prediction model 61B such that temporary second prediction data is output from the temperature preservation stability prediction model 61B. The prediction unit 67 repeats this process on the plurality of types of prepared solutions 35 such that a plurality of temporary second prediction data items are output from the temperature preservation stability prediction model 61B. The prediction unit 67 calculates an average value of the plurality of temporary second prediction data items and sets the calculated average value as the second prediction data 21_2. The temporary second prediction data and thus the second prediction data 21_2 are data that predicts the DSC analysis data 41(4W) in the fourth week in a case where the target solution represented by the second predicted prescription information 18_2 is heated at the preservation temperature 27. In addition, instead of the average value, the second prediction data 21_2 may be the median value, maximum value, or minimum value of the plurality of temporary second prediction data items or may be a weighted average value calculated by multiplying the plurality of temporary second prediction data items by an appropriate weight.

As in the case illustrated in Fig. 19, Fig. 23 illustrates a case where five temporary second prediction data items of No. 1 to No. 5 are derived for five types of prepared solutions 35 of No. 1 to No. 5 in which the type 25 of the additive is any one of L-arginine hydrochloride or L-histidine hydrochloride, the hydrogen ion exponent is any one of 6.5, 7.0, or 7.5, and the preservation temperature 27 is any one of 40°C, 45°C, or 50°C. For the temporary second prediction data, No. 1 and No. 2 are 50, No. 3 is 48, No. 4 is 62, and No. 5 is 60. Therefore, the second prediction data 21_1 is (50 + 50 + 48 + 62 + 60)/5 = 54.

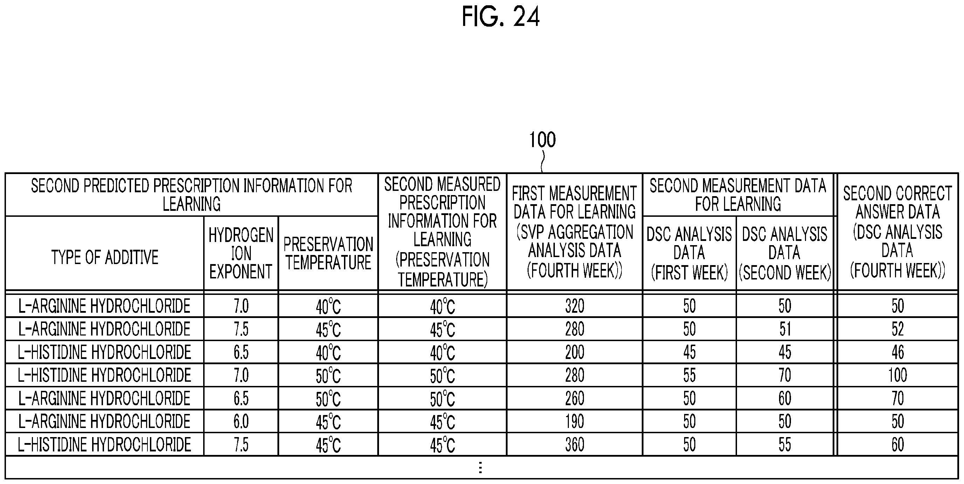

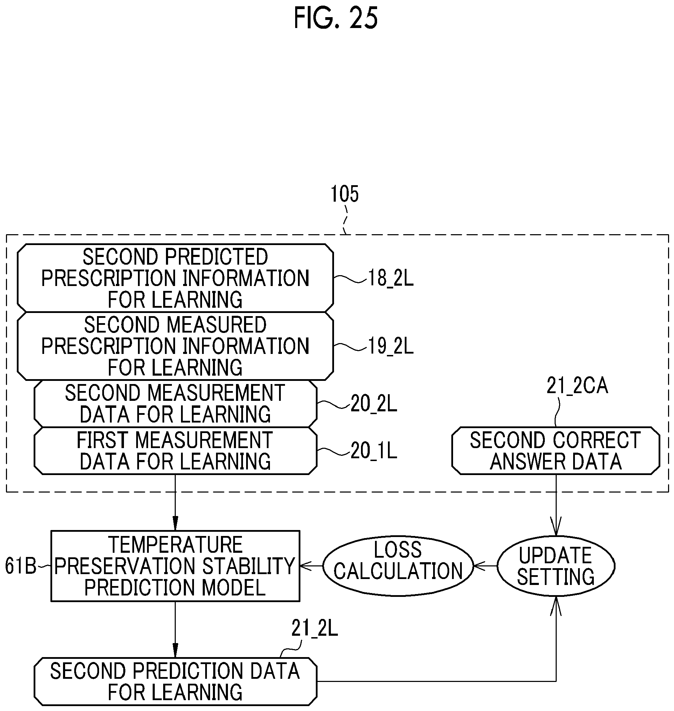

As illustrated in a table 100 of Fig. 24 as an example, as learning data 105 for the temperature preservation stability prediction model 61B, a plurality of sets of second predicted prescription information 18_2L for learning, the preservation temperature 27 of second measured prescription information 19_2L for learning, the first measurement data 20_1L for learning, second measurement data 20_2L for learning, and second correct answer data 21_2CA (see Fig. 25) are stored in, for example, the storage 50. The same content is registered in the preservation temperature 27 of the second predicted prescription information for learning 18_2L and the preservation temperature 27 of the second measured prescription information for learning 19_2L. The first measurement data 20_1L for learning is data obtained by actually measuring the preservation stability of the prepared solution 35 in which the second measured prescription information 19_2L for learning has been registered and is the SVP aggregation analysis data 40(4W) in the fourth week. The first measurement data 20_1L for learning is data corresponding to the first prediction data 21_1 in the operation phase of the temperature preservation stability prediction model 61B illustrated in Figs. 22 and 23. The second measurement data 20_2L for learning is data obtained by actually measuring the preservation stability of the prepared solution 35 in which the second measured prescription information 19_2L for learning has been registered and is the DSC analysis data 41(1W) in the first week and the DSC analysis data 41(2W) in the second week.

Further, similarly, the second correct answer data 21_2CA is data obtained by actually measuring the preservation stability of the prepared solution 35 in which the second measured prescription information 19_2L for learning has been registered. Specifically, the second correct answer data 21_2CA is the DSC analysis data 41(4W) in the fourth week. In addition, in a case where the first measurement data 20_1L for learning is not actually measured, a value prepared in advance or a mask indicating that there is no data is registered. Alternatively, the first prediction data 21_1 predicted by the aggregation preservation stability prediction model 61A may be registered as the first measurement data 20_1L for learning.

As illustrated in Fig. 25 as an example, the learning data 105 is given in a learning phase of the temperature preservation stability prediction model 61B. The learning data 105 is a set of the second predicted prescription information 18_2L for learning, the second measured prescription information 19_2L for learning, the first measurement data 20_1L for learning, the second measurement data 20_2L for learning, and the second correct answer data 21_2CA registered in the table 100 illustrated in Fig. 24. The second predicted prescription information 18_2L for learning, the second measured prescription information 19_2L for learning, the first measurement data 20_1L for learning, and the second measurement data 20_2L for learning in the learning data 105 are input to the temperature preservation stability prediction model 61B. As a result, second prediction data 21_2L for learning is output from the temperature preservation stability prediction model 61B.

Loss calculation of the temperature preservation stability prediction model 61B using the loss function is performed on the basis of the second prediction data 21_2L for learning and the second correct answer data 21_2CA. Then, the update setting of various coefficients of the temperature preservation stability prediction model 61B is performed according to a result of the loss calculation, and the temperature preservation stability prediction model 61B is updated according to the update setting.

In the learning phase of the temperature preservation stability prediction model 61B, the series of processes of the input of the second predicted prescription information 18_2L for learning, the second measured prescription information 19_2L for learning, the first measurement data 20_1L for learning, and the second measurement data 20_2L for learning to the temperature preservation stability prediction model 61B, the output of the second prediction data 21_2L for learning from the temperature preservation stability prediction model 61B, the loss calculation, the update setting, and the update of the temperature preservation stability prediction model 61B is repeated while the learning data 105 is exchanged. The repetition of the series of processes is ended in a case where the prediction accuracy of the second prediction data 21_2L for learning with respect to the second correct answer data 21_2CA has reached a predetermined set level. The temperature preservation stability prediction model 61B in which the prediction accuracy has reached the set level in this manner is stored in the storage 50 and is used by the prediction unit 67. In addition, the learning may be ended in a case where the series of processes is repeated a set number of times, regardless of the prediction accuracy of the second prediction data 21_2L for learning with respect to the second correct answer data 21_2CA.

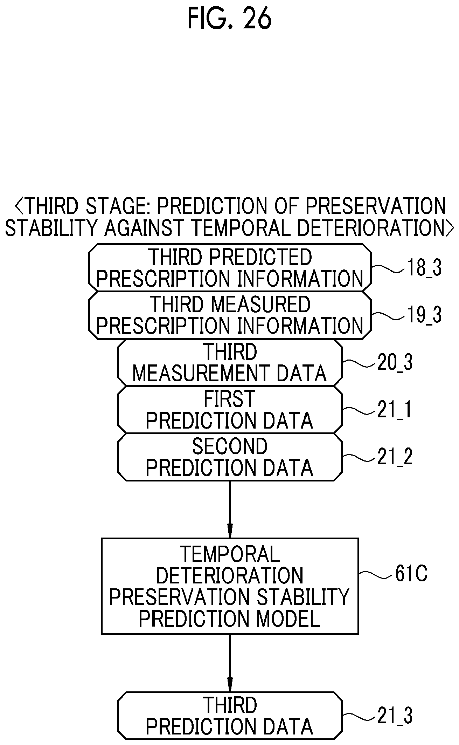

As illustrated in Fig. 26 as an example, in the prediction in the third stage, the prediction unit 67 inputs third predicted prescription information 18_3, the preservation period 28 of third measured prescription information 19_3, third measurement data 20_3, the first prediction data 21_1, and the second prediction data 21_2 to the temporal deterioration preservation stability prediction model 61C. Then, third prediction data 21_3 is output from the temporal deterioration preservation stability prediction model 61C. In addition, similarly to the third predicted prescription information 18_3 and the like, the third prediction data 21_3 is given the solution ID. The prediction in the third stage by the temporal deterioration preservation stability prediction model 61C is an example of the "prediction process in the subsequent stage" according to the technology of the present disclosure. In addition, the prediction in the first stage by the aggregation preservation stability prediction model 61A and the prediction in the second stage by the temperature preservation stability prediction model 61B are examples of the "prediction process in the previous stage" according to the technology of the present disclosure, and the first prediction data 21_1 and the second prediction data 21_2 are examples of the "prediction data obtained in the prediction process in the previous stage" according to the technology of the present disclosure. That is, the prediction in the second stage by the temperature preservation stability prediction model 61B is the "prediction process in the subsequent stage" with respect to the prediction in the first stage and is the "prediction process in the previous stage" with respect to the prediction in the third stage.

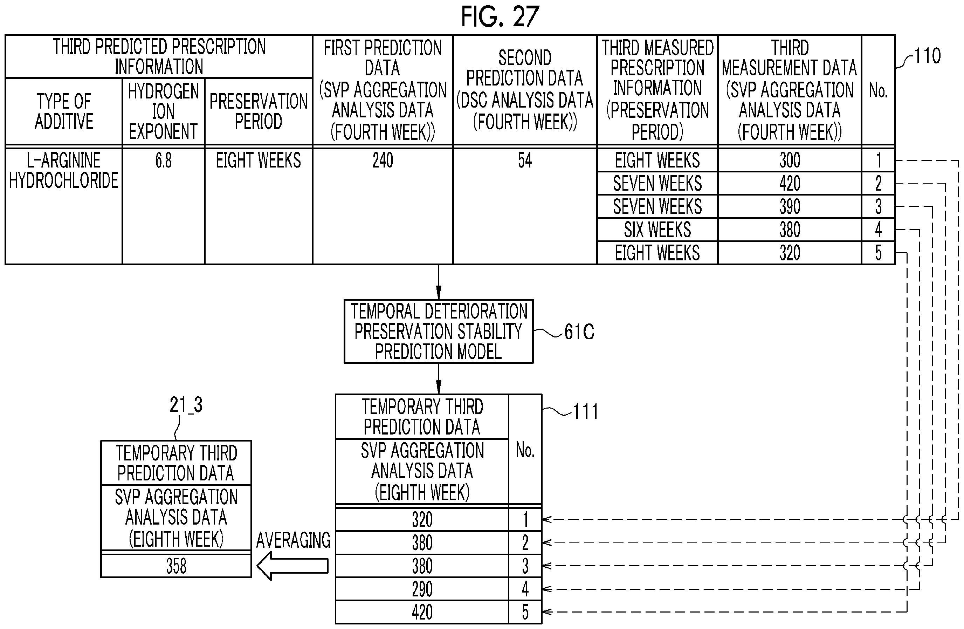

As illustrated in tables 110 and 111 of Fig. 27 as an example, in the prediction in the third stage, the prediction unit 67 inputs the third predicted prescription information 18_3, one of sets of the preservation period 28 of the third measured prescription information 19_3 and the third measurement data 20_3 of a plurality of types of prepared solutions 35, the first prediction data 21_1, and the second prediction data 21_2 to the temporal deterioration preservation stability prediction model 61C such that temporary third prediction data is output from the temporal deterioration preservation stability prediction model 61C. The prediction unit 67 repeats this process on the plurality of types of prepared solutions 35 such that a plurality of temporary third prediction data items are output from the temporal deterioration preservation stability prediction model 61C. The prediction unit 67 calculates an average value of the plurality of temporary third prediction data items and sets the calculated average value as the third prediction data 21_3. The temporary third prediction data and thus the third prediction data 21_3 are data that predicts the SVP aggregation analysis data 40 of the target solution represented by the third predicted prescription information 18_3 in the preservation period 28 (here, in the eighth week). In addition, instead of the average value, the third prediction data 21_3 may be the median value, maximum value, or minimum value of the plurality of temporary third prediction data items or may be a weighted average value calculated by multiplying the plurality of temporary third prediction data items by an appropriate weight.

As in the cases illustrated in Figs. 19 and 23, Fig. 27 illustrates a case where five temporary third prediction data items of No. 1 to No. 5 are derived for five types of prepared solutions 35 of No. 1 to No. 5 in which the type 25 of the additive is any one of L-arginine hydrochloride or L-histidine hydrochloride, the hydrogen ion exponent is any one of 6.5, 7.0, or 7.5, and the preservation period 28 is any one of six weeks, seven weeks, or eight weeks. For the temporary third prediction data, No. 1 is 320, No. 2 and No. 3 are 380, No. 4 is 290, and No. 5 is 420. Therefore, the third prediction data 21_1 is (320 + 380 + 380 + 290 + 420)/5 = 358.

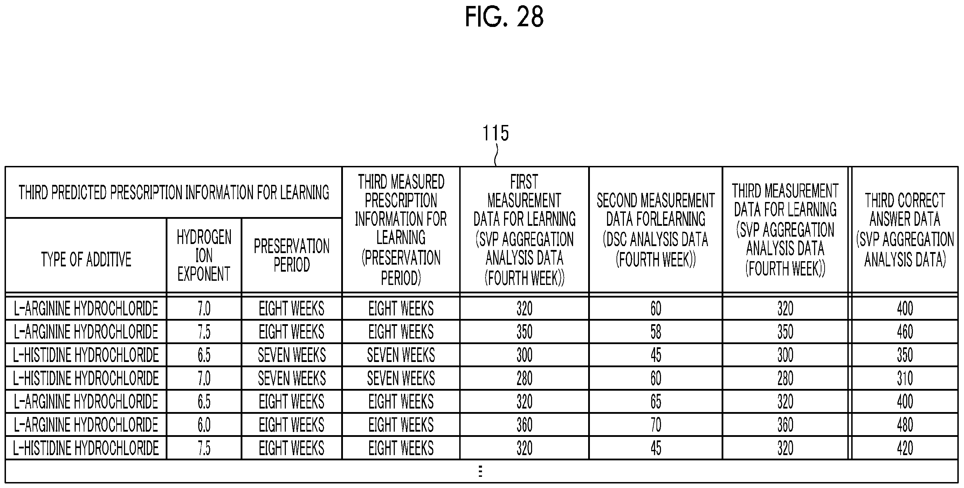

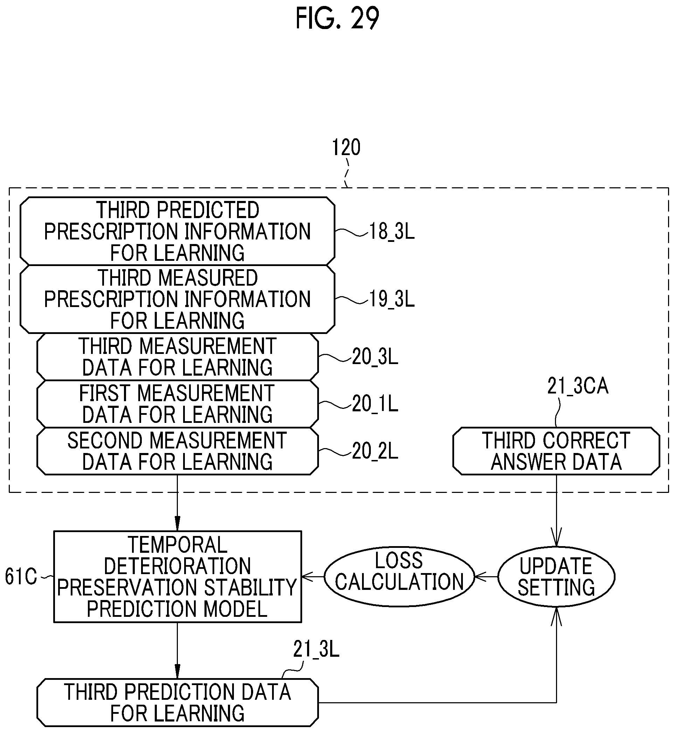

As illustrated in a table 115 illustrated in Fig. 28 as an example, as learning data 120 for the temporal deterioration preservation stability prediction model 61C, a plurality of sets of third predicted prescription information 18_3L for learning, the preservation period 28 of third measured prescription information 19_3L for learning, the first measurement data 20_1L for learning, the second measurement data 20_2L for learning, third measurement data 20_3L for learning, and third correct answer data 21_3CA (see Fig. 29) are stored in, for example, the storage 50. The same content is registered in the preservation period 28 of the third predicted prescription information 18_3L for learning and the preservation period 28 of the third measured prescription information 19_3L for learning. The first measurement data 20_1L for learning is data obtained by actually measuring the preservation stability of the prepared solution 35 in which the third measured prescription information 19_3L for learning has been registered and is the SVP aggregation analysis data 40(4W) in the fourth week. The first measurement data 20_1L for learning is data corresponding to the first prediction data 21_1 in the operation phase of the temporal deterioration preservation stability prediction model 61C illustrated in Figs. 26 and 27. The second measurement data 20_2L for learning is data obtained by actually measuring the preservation stability of the prepared solution 35 in which the third measured prescription information 19_3L for learning has been registered and is the DSC analysis data 41(4W) in the fourth week. The second measurement data 20_2L for learning is data corresponding to the second prediction data 21_2 in the operation phase of the temporal deterioration preservation stability prediction model 61C illustrated in Figs. 26 and 27.

Similarly, the third correct answer data 21_3CA is data obtained by actually measuring the preservation stability of the prepared solution 35 in which the third measured prescription information 19_3L for learning has been registered. Specifically, the third correct answer data 21_3CA is the SVP aggregation analysis data 40 in the preservation period 28 of the third measured prescription information 19_3L for learning. In addition, in a case where the first measurement data 20_1L for learning is not actually measured, a value prepared in advance or a mask indicating that there is no data is registered. Alternatively, the first prediction data 21_1 predicted by the aggregation preservation stability prediction model 61A may be registered as the first measurement data 20_1L for learning. Similarly, in a case where the second measurement data 20_2L for learning is not actually measured, a value prepared in advance or a mask indicating that there is no data may be registered, or the second prediction data 21_2 predicted by the temperature preservation stability prediction model 61B may be registered as the second measurement data 20_2L for learning.

As illustrated in Fig. 29 as an example, the learning data 120 is given in a learning phase of the temporal deterioration preservation stability prediction model 61C. The learning data 120 is a set of the third predicted prescription information 18_3L for learning, the third measured prescription information 19_3L for learning, the first measurement data 20_1L for learning, the second measurement data 20_2L for learning, the third measurement data 20_3L for learning, and the third correct answer data 21_3CA registered in the table 115 illustrated in Fig. 28. The third predicted prescription information 18_3L for learning, the third measured prescription information 19_3L for learning, the first measurement data 20_1L for learning, the second measurement data 20_2L for learning, and the third measurement data 20_3L for learning in the learning data 120 are input to the temporal deterioration preservation stability prediction model 61C. As a result, third prediction data 21_3L for learning is output from the temporal deterioration preservation stability prediction model 61C.

Loss calculation of the temporal deterioration preservation stability prediction model 61C using the loss function is performed on the basis of the third prediction data 21_3L for learning and the third correct answer data 21_3CA. Then, the update setting of various coefficients of the temporal deterioration preservation stability prediction model 61C is performed according to a result of the loss calculation, and the temporal deterioration preservation stability prediction model 61C is updated according to the update setting.

In the learning phase of the temporal deterioration preservation stability prediction model 61C, the series of processes of the input of the third predicted prescription information 18_3L for learning, the third measured prescription information 19_3L for learning, the first measurement data 20_1L for learning, the second measurement data 20_2L for learning, and the third measurement data 20_3L for learning to the temporal deterioration preservation stability prediction model 61C, the output of the third prediction data 21_3L for learning from the temporal deterioration preservation stability prediction model 61C, the loss calculation, the update setting, and the update of the temporal deterioration preservation stability prediction model 61C is repeated while the learning data 120 is exchanged. The repetition of the series of processes is ended in a case where the prediction accuracy of the third prediction data 21_3L for learning with respect to the third correct answer data 21_3CAhas reached a predetermined set level. The temporal deterioration preservation stability prediction model 61C in which the prediction accuracy has reached the set level in this manner is stored in the storage 50 and is used by the prediction unit 67. In addition, the learning may be ended in a case where the series of processes is repeated a set number of times, regardless of the prediction accuracy of the third prediction data 21_3L for learning with respect to the third correct answer data 21_3CA.

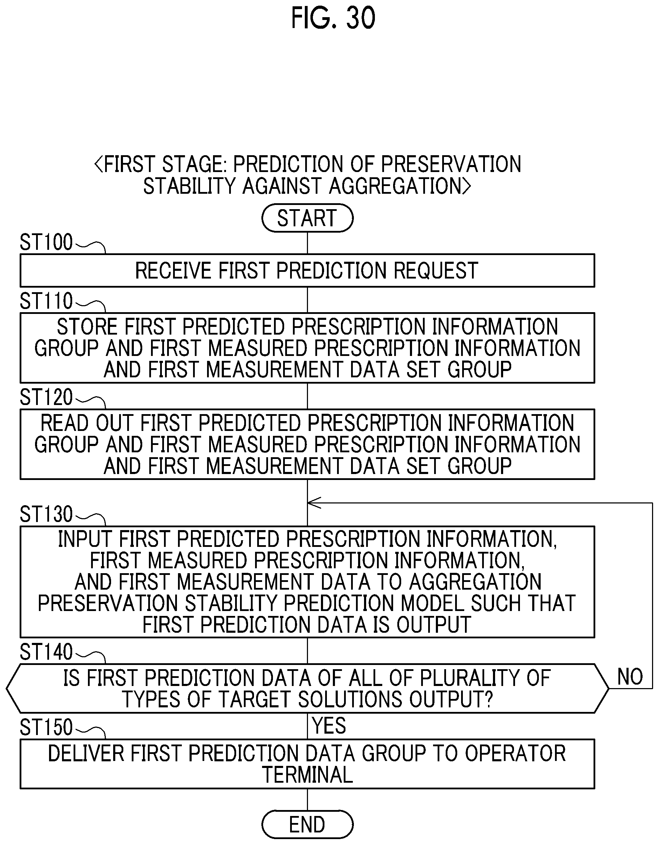

Next, the operation of the above-described configuration will be described with reference to flowcharts illustrated in Figs. 30, 31, and 32. First, in a case where the operation program 60 is started in the pharmaceutical support server 10, the CPU 52 of the pharmaceutical support server 10 functions as the receiving unit 65, the RW control unit 66, the prediction unit 67, and the delivery control unit 68 as illustrated in Fig. 16.

The operator inputs the desired first predicted prescription information 18_1, first measured prescription information 19_1, and first measurement data 20_1 through the input device 14 in order to predict the preservation stability of the antibody 37 against aggregation in the first stage. Then, the first prediction request 15_1 including the first predicted prescription information group 16_1 and the first measured prescription information and first measurement data set group 17_1 is transmitted from the operator terminal 11 to the pharmaceutical support server 10.

As illustrated in Fig. 30 as an example, in the pharmaceutical support server 10, the first prediction request 15_1 is received by the receiving unit 65. Therefore, the first predicted prescription information group 16_1 and the first measured prescription information and first measurement data set group 17_1 are acquired (Step ST100). The first predicted prescription information group 16_1 and the first measured prescription information and first measurement data set group 17_1 are output from the receiving unit 65 to the RW control unit 66 and are stored in the storage 50 under the control of the RW control unit 66 (Step ST110).

The RW control unit 66 reads out the first predicted prescription information group 16_1 and the first measured prescription information and first measurement data set group 17_1 from the storage 50 (Step ST120). The first predicted prescription information group 16_1 and the first measured prescription information and first measurement data set group 17_1 are output from the RW control unit 66 to the prediction unit 67.

As illustrated in Figs. 18 and 19, the prediction unit 67 inputs the first predicted prescription information 18_1, the first measured prescription information 19_1, and the first measurement data 20_1 to the aggregation preservation stability prediction model 61A. As a result, the first prediction data 21_1 is output from the aggregation preservation stability prediction model 61A (Step ST130). The first prediction data 21_1 is output from the prediction unit 67 to the delivery control unit 68. The process in Step ST130 is repeatedly continued while the first prediction data 21_1 of all of a plurality of types of target solutions is not output (NO in Step ST140).

In a case where the first prediction data 21_1 of all of the plurality of types of target solutions is output (YES in Step ST140), the first prediction data group 22_1 is delivered to the operator terminal 11 which is the transmission source of the first prediction request 15_1 under the control of the delivery control unit 68 (Step ST150).

The first prediction data group 22_1 is displayed on the display 13 of the operator terminal 11. The operator narrows the plurality of target solutions predicted in the first stage down to the target solutions to proceed to the prediction in the second stage on the basis of the first prediction data group 22_1.

Then, the operator inputs the desired second predicted prescription information 18_2, second measured prescription information 19_2, and second measurement data 20_2 through the input device 14 in order to predict the preservation stability of the antibody 37 against temperature in the second stage. Then, the second prediction request 15_2 including the second predicted prescription information group 16_2, the second measured prescription information and second measurement data set group 17_2, and the first prediction data group 22_1 obtained in the prediction in the first stage is transmitted from the operator terminal 11 to the pharmaceutical support server 10.

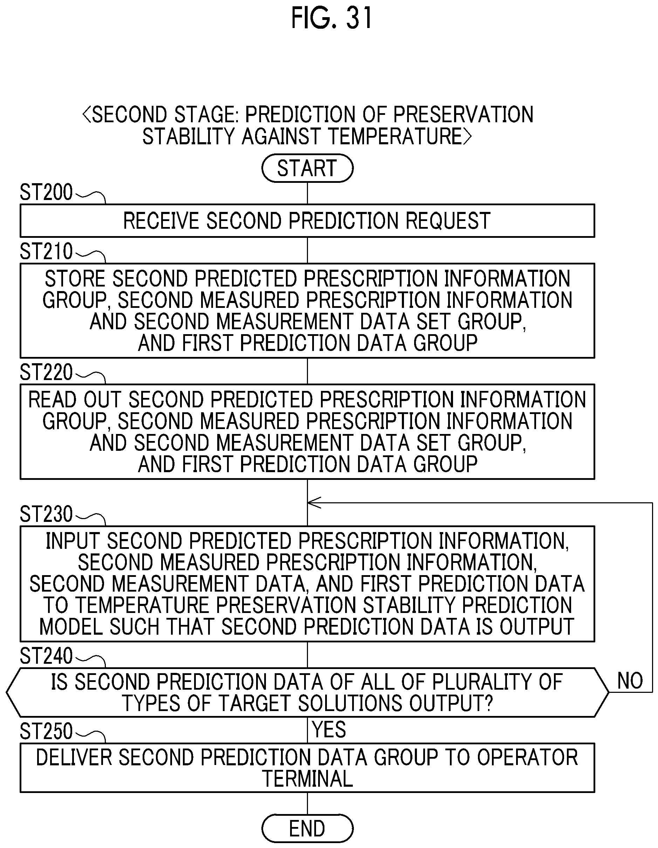

As illustrated in Fig. 31 as an example, in the pharmaceutical support server 10, the second prediction request 15_2 is received by the receiving unit 65. Therefore, the second predicted prescription information group 16_2, the second measured prescription information and second measurement data set group 17_2, and the first prediction data group 22_1 are acquired (Step ST200). The second predicted prescription information group 16_2, the second measured prescription information and second measurement data set group 17_2, and the first prediction data group 22_1 are output from the receiving unit 65 to the RW control unit 66 and are stored in the storage 50 under the control of the RW control unit 66 (Step ST210).

The RW control unit 66 reads out the second predicted prescription information group 16_2, the second measured prescription information and second measurement data set group 17_2, and the first prediction data group 22_1 from the storage 50 (Step ST220). The second predicted prescription information group 16_2, the second measured prescription information and second measurement data set group 17_2, and the first prediction data group 22_1 are output from the RW control unit 66 to the prediction unit 67.

As illustrated in Figs. 22 and 23, in the prediction unit 67, the second predicted prescription information 18_2, the preservation temperature 27 of the second measured prescription information 19_2, the second measurement data 20_2, and the first prediction data 21_1 are input to the temperature preservation stability prediction model 61B. As a result, the second prediction data 21_2 is output from the temperature preservation stability prediction model 61B (Step ST230). The second prediction data 21_2 is output from the prediction unit 67 to the delivery control unit 68. The process in Step ST230 is repeatedly continued while the second prediction data 21_2 of all of the plurality of types of target solutions is not output (NO in Step ST240).

In a case where the second prediction data 21_2 of all of the plurality of types of target solutions is output (YES in Step ST240), the second prediction data group 22_2 is delivered to the operator terminal 11 which is the transmission source of the second prediction request 15_2 under the control of the delivery control unit 68 (Step ST250).

The second prediction data group 22_2 is displayed on the display 13 of the operator terminal 11. The operator narrows the plurality of target solutions predicted in the second stage down to the target solutions to proceed to the prediction in the third stage on the basis of the second prediction data group 22_2.

Then, the operator inputs the desired third predicted prescription information 18_3, third measured prescription information 19_3, and third measurement data 20_3 through the input device 14 in order to predict the preservation stability of the antibody 37 against temporal deterioration in the third stage. Then, the third prediction request 15_3 including the third predicted prescription information group 16_3, the third measured prescription information and third measurement data set group 17_3, the first prediction data 21_1 obtained in the prediction in the first stage, and the second prediction data 21_2 obtained in the prediction in the second stage is transmitted from the operator terminal 11 to the pharmaceutical support server 10.

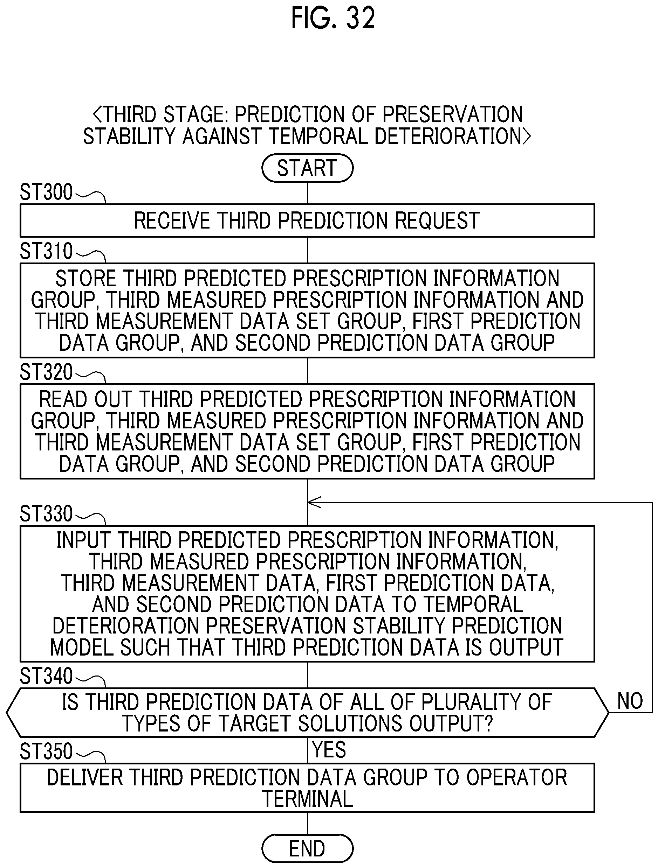

As illustrated in Fig. 32 as an example, in the pharmaceutical support server 10, the third prediction request 15_3 is received by the receiving unit 65. Therefore, the third predicted prescription information group 16_3, the third measured prescription information and third measurement data set group 17_3, the first prediction data group 22_1, and the second prediction data group 22_2 are acquired (Step ST300). The third predicted prescription information group 16_3, the third measured prescription information and third measurement data set group 17_3, the first prediction data group 22_1, and the second prediction data group 22_2 are output from the receiving unit 65 to the RW control unit 66 and are stored in the storage 50 under the control of the RW control unit 66 (Step ST310).

The RW control unit 66 reads the third predicted prescription information group 16_3, the third measured prescription information and third measurement data set group 17_3, the first prediction data group 22_1, and the second prediction data group 22_2 from the storage 50 (Step ST320). The third predicted prescription information group 16_3, the third measured prescription information and third measurement data set group 17_3, the first prediction data group 22_1, and the second prediction data group 22_2 are output from the RW control unit 66 to the prediction unit 67.

As illustrated in Figs. 26 and 27, in the prediction unit 67, the third predicted prescription information 18_3, the preservation period 28 of the third measured prescription information 19_3, the third measurement data 20_3, the first prediction data 21_1, and the second prediction data 21_2 are input to the temporal deterioration preservation stability prediction model 61C. As a result, the third prediction data 21_3 is output from the temporal deterioration preservation stability prediction model 61C (Step ST330). The third prediction data 21_3 is output from the prediction unit 67 to the delivery control unit 68. The process in Step ST330 is repeatedly continued while the third prediction data 21_3 of all of the plurality of types of target solutions is not output (NO in Step ST340).

In a case where the third prediction data 21_3 of all of the plurality of types of target solutions is output (YES in Step ST340), the third prediction data group 22_3 is delivered to the operator terminal 11 which is the transmission source of the third prediction request 15_3 under the control of the delivery control unit 68 (Step ST350).

The third prediction data group 22_3 is displayed on the display 13 of the operator terminal 11. The operator selects a target solution to be adopted as the preservation solution for the biopharmaceutical from the plurality of target solutions predicted in the second stage, on the basis of the third prediction data group 22_3.

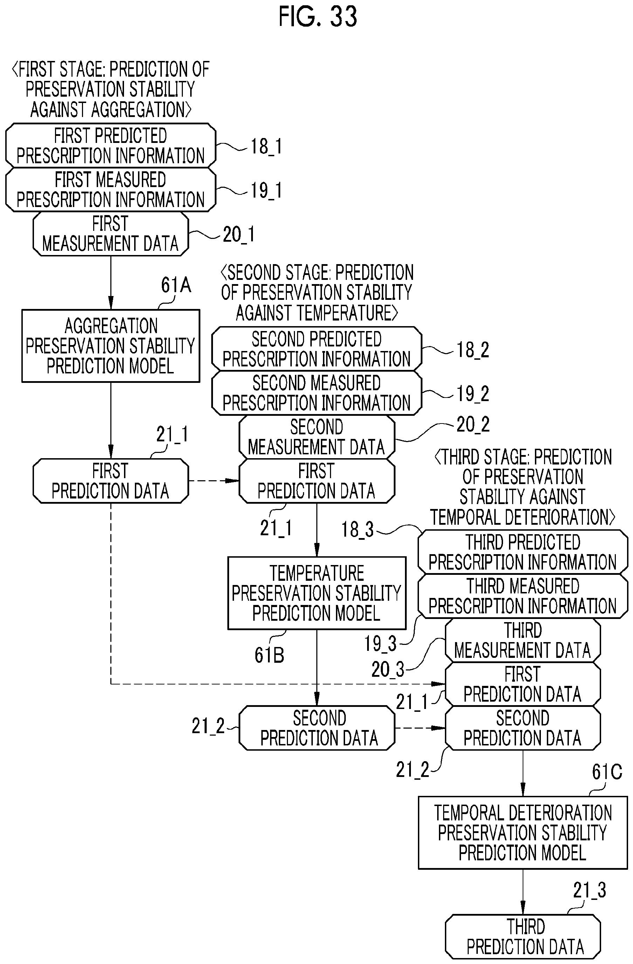

As described above, the prediction unit 67 of the CPU 52 of the pharmaceutical support server 10 uses the preservation stability prediction model 61 that outputs the prediction data 21 indicating the preservation stability of the candidate preservation solution, which is a candidate for the preservation solution for the biopharmaceutical, at a future time point and that is provided for each of a plurality of types of preservation stability. Specifically, as illustrated in Fig. 33, the prediction unit 67 uses the aggregation preservation stability prediction model 61A that outputs the first prediction data 21_1 indicating the preservation stability of the antibody 37 against aggregation, the temperature preservation stability prediction model 61B that outputs the second prediction data 21_2 indicating the preservation stability of the antibody 37 against temperature, and the temporal deterioration preservation stability prediction model 61C that outputs the third prediction data 21_3 indicating the preservation stability of the antibody 37 against temporal deterioration 37.

The prediction unit 67 performs in stages the prediction process of inputting the predicted prescription information 18 related to the prescription of the target solution and the measurement data 20 obtained by actually measuring the preservation stability of the prepared solution 35 to the preservation stability prediction model 61 such that the prediction data 21 is output from the preservation stability prediction model 61. Specifically, as illustrated in Fig. 33, in the prediction in the first stage, the first predicted prescription information 18_1 and the first measurement data 20_1 are input to the aggregation preservation stability prediction model 61A such that the first prediction data 21_1 is output from the aggregation preservation stability prediction model 61A. Then, in the prediction in the second stage, the second predicted prescription information 18_2 and the second measurement data 20_2 are input to the temperature preservation stability prediction model 61B such that the second prediction data 21_2 is output from the temperature preservation stability prediction model 61B. Finally, in the prediction in the third stage, the third predicted prescription information 18_3 and the third measurement data 20_3 are input to the temporal deterioration preservation stability prediction model 61C such that the third prediction data 21_3 is output from the temporal deterioration preservation stability prediction model 61C.

Further, the prediction unit 67 inputs the prediction data 21 obtained in the prediction process in the previous stage to the preservation stability prediction model 61 in the prediction process in the subsequent stage. Specifically, as illustrated in Fig. 33, the first prediction data 21_1 obtained in the prediction in the first stage is input to the temperature preservation stability prediction model 61B in the prediction in the second stage. Further, the first prediction data 21_1 obtained in the prediction in the first stage and the second prediction data 21_2 obtained in the prediction in the second stage are input to the temporal deterioration preservation stability prediction model 61C in the prediction in the third stage.

Therefore, as compared to a case where a plurality of types of preservation stability are collectively predicted in the form of integrated preservation stability and a case where the plurality of types of preservation stability are individually predicted, it is possible to reduce a concern that it will not be possible to successfully search for the prescription of the preservation solution suitable for the biopharmaceutical.

It is not possible to prepare a large amount of learning data 90 illustrated in the table 85 of Fig. 20, a large amount of learning data 105 illustrated in the table 100 of Fig. 24, and a large amount of learning data 120 illustrated in the table 115 of Fig. 28. In addition, as described above, the preservation stability of the preservation solution varies widely. For this reason, it is difficult to collectively predict a plurality of types of preservation stability using one preservation stability prediction model. Therefore, the use of the technology of the present disclosure that divides the preservation stability to be predicted and predicts the preservation stability in stages makes it possible to more stably and accurately predict a plurality of types of preservation stability with a small amount of learning data.

In addition, for example, it is possible to use a method that narrows down the candidate preservation solutions in stages in such a way that 1,000 target solutions are narrowed down to 100 target solutions in the first stage, the 100 target solutions are narrowed down to 10 target solutions in the second stage, and one target solution to be adopted as the preservation solution for the biopharmaceutical is selected from the 10 target solutions in the third stage. According to this method, it is possible to increase the probability that the prescription of the preservation solution will be suitable for the biopharmaceutical, as compared to a case where one target solution to be adopted as the preservation solution for the biopharmaceutical is selected from, for example, 1,000 target solutions with one evaluation criterion.

As illustrated in Fig. 18 and the like, the prediction unit 67 also inputs the measured prescription information 19 of the prepared solution 35 to the preservation stability prediction model 61. Therefore, it is possible to improve the prediction accuracy of the prediction data 21. In addition, as illustrated in Fig. 19 and the like, in a case where the measured prescription information 19 of a plurality of types of prepared solutions 35 is input, it is possible to obtain the prediction data 21 with high prediction accuracy even though unknown content (for example, a hydrogen ion exponent of 6.8 in Figure 19 or a preservation temperature 27 of 42°C in Fig. 23) is input as the predicted prescription information 18 of the target solution that has not actually been prepared. Therefore, it is possible to reduce the time and effort required to actually prepare the target solution. As a result, it is possible to promote the development of biopharmaceuticals.

The measurement data 20 is time-series data measured at at least two time points like the SVP aggregation analysis data 40(1W) in the first week and the SVP aggregation analysis data 40(2W) in the second week in the first measurement data 20_1 illustrated in Fig. 12 and the DSC analysis data 41(1W) in the first week and the DSC analysis data 41(2W) in the second week in the second measurement data 20_2 illustrated in Fig. 13. Therefore, it is possible to improve the prediction accuracy of the first prediction data 21_1 and the second prediction data 21_2 as compared to a case where the measurement data 20 is not time-series data.

As illustrated in Fig. 5 and the like, the predicted prescription information 18 includes the type 25 of the additive and the hydrogen ion exponent 26. Therefore, it is possible to select a preservation solution in which at least the type 25 of the additive and the hydrogen ion exponent 26 are suitable for the biopharmaceutical.

As illustrated in Fig. 12 and the like, the measurement data 20 includes the SVP aggregation analysis data 40 and the DSC analysis data 41 of the antibody 37 in the prepared solution 35. Therefore, the prediction data 21 that more accurately indicates the preservation stability of the prepared solution 35 can be output from the preservation stability prediction model 61.

The biopharmaceutical including the antibody 37 as the protein is called an antibody drug and is widely used not only for the treatment of chronic diseases, such as cancer, diabetes, and rheumatoid arthritis, but also for the treatment of rare diseases such as hemophilia and a Crohn's disease. Therefore, according to the example in which the protein is the antibody 37, it is possible to further promote the development of antibody drugs widely used for the treatment of various diseases.

The training of the aggregation preservation stability prediction model 61A illustrated in Fig. 21, the training of the temperature preservation stability prediction model 61B illustrated in Fig. 25, and the training of the temporal deterioration preservation stability prediction model 61C illustrated in Fig. 29 may be performed by the pharmaceutical support server 10 or may be performed by a device other than the pharmaceutical support server 10. In addition, the aggregation preservation stability prediction model 61A, the temperature preservation stability prediction model 61B, and the temporal deterioration preservation stability prediction model 61C may be continuously trained even after being stored in the storage 50.

### [Second Embodiment]

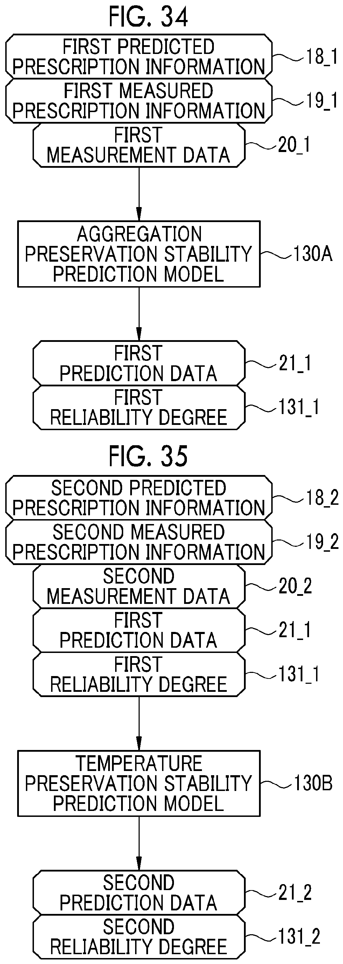

In a second embodiment illustrated in Figs. 34 to 37 as an example, a reliability degree of the prediction data 21 is also input to the preservation stability prediction model.

As illustrated in Fig. 34 as an example, in a case where the first predicted prescription information 18_1, the first measured prescription information 19_1, and the first measurement data 20_1 are input to an aggregation preservation stability prediction model 130A according to the second embodiment, the aggregation preservation stability prediction model 130A outputs a first reliability degree 131_1 which is a reliability degree of the prediction of the first prediction data 21_1, in addition to the first prediction data 21_1. The first reliability degree 131_1 has a value between 0 and 1. A case where the first reliability degree 131_1 is 0 indicates that the reliability degree of the prediction of the first prediction data 21_1 is the lowest value, and a case where the first reliability degree 131_1 is 1 indicates that the reliability degree of the prediction of the first prediction data 21_1 is the highest value.

As illustrated in Fig. 35 as an example, in the second embodiment, in the prediction unit 67, in addition to the second predicted prescription information 18_2, the second measured prescription information 19_2, the second measurement data 20_2, and the first prediction data 21_1 obtained in the prediction in the first stage, the first reliability degree 131_1 obtained in the prediction in the first stage is input to a temperature preservation stability prediction model 130B. In a case where the second predicted prescription information 18_2, the second measured prescription information 19_2, the second measurement data 20_2, the first prediction data 21_1, and the first reliability degree 131_1 are input to the temperature preservation stability prediction model 130B, the temperature preservation stability prediction model 130B outputs a second reliability degree 131_2 which is a reliability degree of the prediction of the second prediction data 21_2, in addition to the second prediction data 21_2. The second reliability degree 131_2 has a value between 0 and 1, similarly to the first reliability degree 131_1. A case where the second reliability degree 131_2 is 0 indicates that the reliability degree of the prediction of the second prediction data 21_2 is the lowest value, and a case where the second reliability degree 131_2 is 1 indicates that the reliability degree of the prediction of the second prediction data 21_2 is the highest value. The first reliability degree 131_1 is an example of a "reliability degree obtained in the prediction process in the previous stage" according to the technology of the present disclosure.

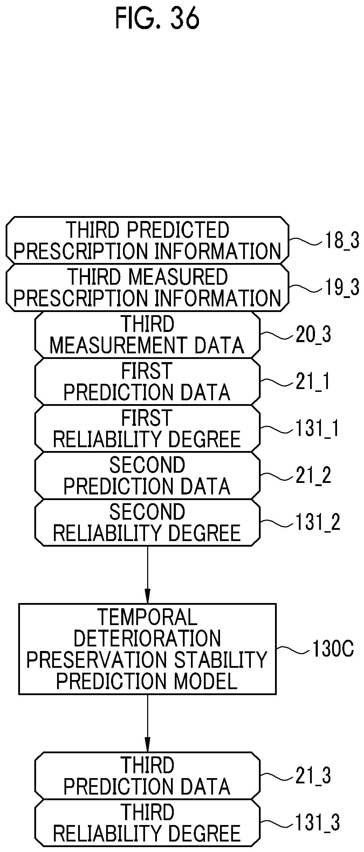

Further, as illustrated in Fig. 36 as an example, in the second embodiment, in the prediction unit 67, in addition to the third predicted prescription information 18_3, the third measured prescription information 19_3, the third measurement data 20_3, the first prediction data 21_1 obtained in the prediction in the first stage, and the second prediction data 21_2 obtained in the prediction in the second stage, the first reliability degree 131_1 obtained in the prediction in the first stage and the second reliability degree 131_2 obtained in the prediction in the second stage are input to a temporal deterioration preservation stability prediction model 130C. In a case where the third predicted prescription information 18_3, the third measured prescription information 19_3, the third measurement data 20_3, the first prediction data 21_1, the first reliability degree 131_1, the second prediction data 21_2, and the second reliability degree 131_2 are input to the temporal deterioration preservation stability prediction model 130C, the temporal deterioration preservation stability prediction model 130C outputs a third reliability degree 131_3 which is a reliability degree of the prediction of the third prediction data 21_3, in addition to the third prediction data 21_3. The third reliability degree 131_3 has a value between 0 and 1 similarly to the first reliability degree 131_1 and the second reliability degree 131_2. A case where the third reliability degree 131_3 is 0 indicates that the reliability degree of the prediction of the third prediction data 21_3 is the lowest value, and a case where the third reliability degree 131_3 is 1 indicates that the reliability degree of the prediction of the third prediction data 21_3 is the highest value. The first reliability degree 131_1 and the second reliability degree 131_2 are examples of the "reliability degree obtained in the prediction process in the previous stage" according to the technology of the present disclosure.

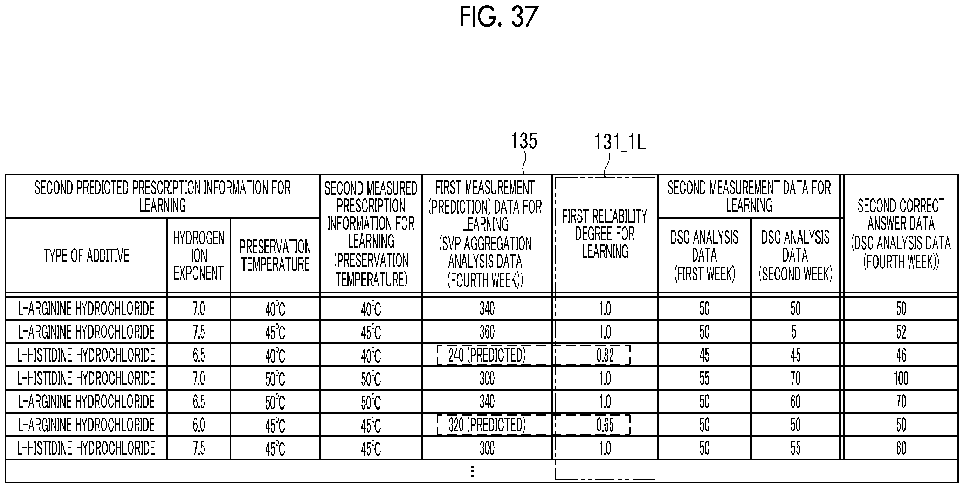

As in a table 135 illustrated in Fig. 37 as an example, in addition to each item such as the second predicted prescription information 18_2L for learning in the learning data 105 for the temperature preservation stability prediction model 61B according to the first embodiment, an item of a first reliability degree 131_1L for learning is added to learning data for the temperature preservation stability prediction model 130B illustrated in Fig. 35. For the first measurement data 20_1L for learning which has been actually measured, the highest value of 1.0 is registered as the first reliability degree 131_1L for learning. In a case where the first measurement data 20_1L for learning is not actually measured and the first prediction data 21_1 predicted by the aggregation preservation stability prediction model 130A is registered as the first measurement data 20_1L for learning as surrounded by a broken line, the first reliability degree 131_1 output by the aggregation preservation stability prediction model 130A is registered as the first reliability degree 131_1L for learning. The items of the first reliability degree for learning and the second reliability degree for learning are added to learning data for the temporal deterioration preservation stability prediction model 130C illustrated in Fig. 36, which is not illustrated. Then, in a case where the first prediction data 21_1 predicted by the aggregation preservation stability prediction model 130A is registered as the first measurement data 20_1L for learning, the first reliability degree 131_1 is registered as the first reliability degree for learning. In a case where the second prediction data 21_2 predicted by the temperature preservation stability prediction model 130B is registered as the second measurement data 20_2L for learning, the second reliability degree 131_2 is registered as the second reliability degree for learning. In addition, for the first prediction data 21_1 predicted by the aggregation preservation stability prediction model 130A and the like, the same value, such as 0.5, may be registered as the first reliability degree for learning and the like.

As described above, in the second embodiment, the aggregation preservation stability prediction model 130A and the temperature preservation stability prediction model 130B output the first reliability degree 131_1 and the second reliability degree 131_2 in addition to the first prediction data 21_1 and the second prediction data 21_2, respectively. The prediction unit 67 also inputs the first reliability degree 131_1 to the temperature preservation stability prediction model 130B and also inputs the first reliability degree 131_1 and the second reliability degree 131_2 to the temporal deterioration preservation stability prediction model 130C. Therefore, it is possible to output the prediction data 21, in which the reliability degree 131 of the prediction data 21 obtained in the prediction process in the previous stage has been added, in the prediction process in the subsequent stage.

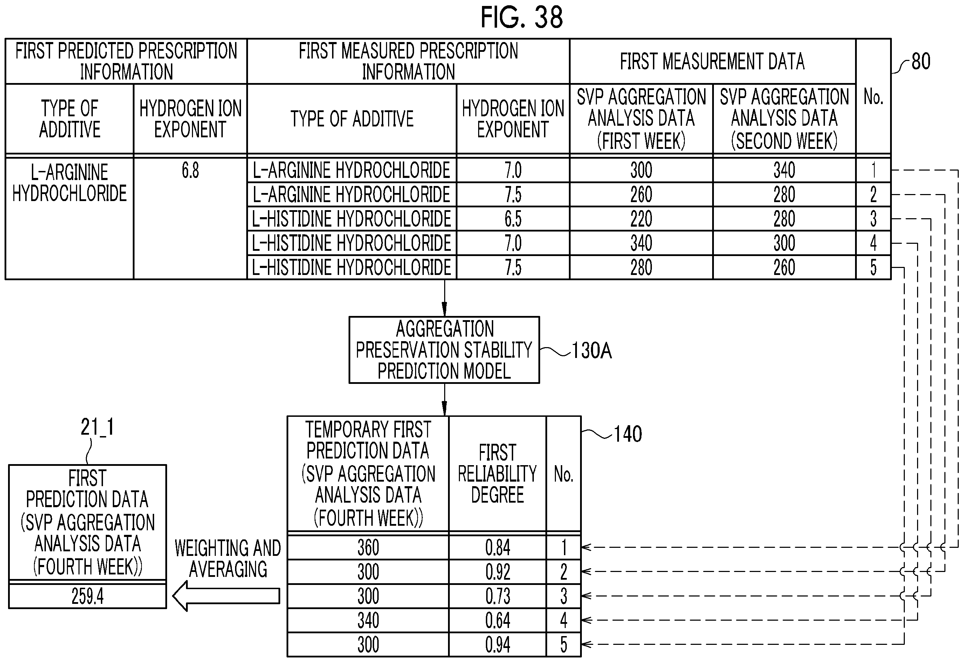

As illustrated in Fig. 38 as an example, a weighted average of a plurality of temporary first prediction data items illustrated in a table 140 output from the aggregation preservation stability prediction model 130A may be calculated using the first reliability degree 131_1 as a weight, and the calculated weighted average value may be used as the first prediction data 21_1. In this case, it is possible to derive the first prediction data 21_1 in which the first reliability degree 131_1 has been reflected. In addition, a weighted average of a plurality of temporary second prediction data items output from the temperature preservation stability prediction model 130B may be calculated using the second reliability degree 131_2 as a weight, and the calculated weighted average value may be used as the second prediction data 21_2, which is not illustrated. Further, a weighted average of a plurality of temporary third prediction data items output from the temporal deterioration preservation stability prediction model 130C may be calculated using the third reliability degree 131_3 as a weight, and the calculated weighted average value may be used as the third prediction data 21_3.

Instead of the weighted average, temporary prediction data having the highest reliability degree 131 may be simply used as the prediction data 21.

### [Third Embodiment]

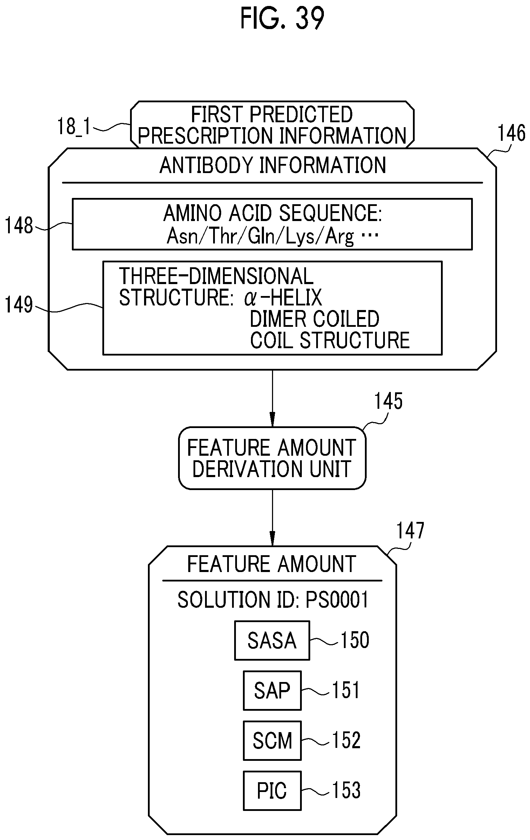

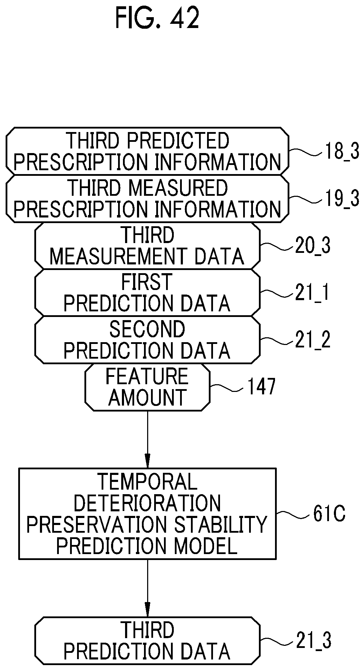

In a third embodiment illustrated in Figs. 39 to 42, the preservation stability prediction model 61 is configured to receive, as an input, antibody information 146 related to the antibody 37 and a feature amount 147 derived on the basis of the antibody information 146. In this case, in the training of the preservation stability prediction model 61, the antibody information 146 and the feature amount 147 are added as learning data together with the predicted prescription information 18L for learning, the measured prescription information 19L for learning, and the like.

As illustrated in Fig. 39 as an example, the CPU of the pharmaceutical support server according to the third embodiment functions as a feature amount derivation unit 145 in addition to the processing units 65 to 68 according to the first embodiment. The feature amount derivation unit 145 derives the feature amount 147 on the basis of the first predicted prescription information 18_1 and the antibody information 146.

The antibody information 146 includes an amino acid sequence 148 of the antibody 37 and a three-dimensional structure 149 of the antibody 37. The antibody information 146 is obtained by analyzing the antibody 37 with a well-known technique such as mass spectrometry, X-ray crystal structure analysis, or electron microscopy. In the amino acid sequence 148, the order of peptide bonds of amino acids constituting the antibody 37, such as asparagine (abbreviated to ASn), glutamine (abbreviated to Glu), and arginine (abbreviated to Arg), is described from an amino terminal to a carboxyl terminal. The amino acid sequence 148 is also referred to as a primary structure. The three-dimensional structure 149 indicates the secondary structure, tertiary structure, and quaternary structure of the amino acids constituting the antibody 37. Examples of the secondary structure include a β-sheet and a β-turn in addition to α-helix given as an example. The tertiary structure is, for example, a dimer coiled coil structure given as an example. Examples of the quaternary structure include a dimer, a trimer, and a tetramer. Some antibodies 37 do not have the quaternary structure (that is, some antibodies 37 are monomers). In this case, the quaternary structure is not registered as described in the example. The antibody information 146 is an example of "protein information" according to the technology of the present disclosure.

The feature amount derivation unit 145 derives, as the feature amount 147, a hydrophobic solvent accessible surface area (hereinafter, abbreviated to SASA) 150, a spatial aggregation propensity (hereinafter, abbreviated to SAP) 151, and a spatial charge map (hereinafter, abbreviated to SCM) 152 of the antibody 37. As the SASA 150 is larger and the SAP 151 is larger, the stability of the antibody 37 is lower. The SCM 152 indicates the degree of charge of the antibody 37 for each region of the antibody 37. The SASA 150, the SAP 151, and the SCM 152 are derived using a molecular dynamics method. In addition, the SASA 150, the SAP 151, and the SCM 152 are derived on the basis of only the antibody information 146 without referring to the first predicted prescription information 18_1. Therefore, the SASA 150, the SAP 151, and the SCM 152 are common to each target solution.

Further, the feature amount derivation unit 145 also derives, as the feature amount 147, a preferential interaction coefficient (hereinafter, abbreviated to PIC) 153 described in Document 1. The PIC 153 indicates, for each region of the antibody 37, the ease of the binding between the antibody 37 and the additive, more specifically, the degree to which the surface of the antibody 37 is covered by the additive. Therefore, the PIC 153 is an indicator showing the compatibility between the antibody 37 and the additive. The PIC 153 makes it possible to know the possibility that the aggregation of the antibody 37 causing a reduction in the drug efficacy of the biopharmaceutical will occur. The PIC 153 is derived on the basis of both the first predicted prescription information 18_1 and the antibody information 146 unlike the SASA 150, the SAP 151, and the SCM 152. Therefore, the PIC 153 is different in each target solution. Therefore, a plurality of feature amounts 147 of each target solution have the SASA 150, the SAP 151, and the SCM 152 in common and have different PICs 153. The PIC 153 may be derived using a machine learning model, such as a support vector machine (SVM), instead of the molecular dynamics method. In addition, the feature amount 147 is given the solution ID, similarly to the first predicted prescription information 18_1 and the like.

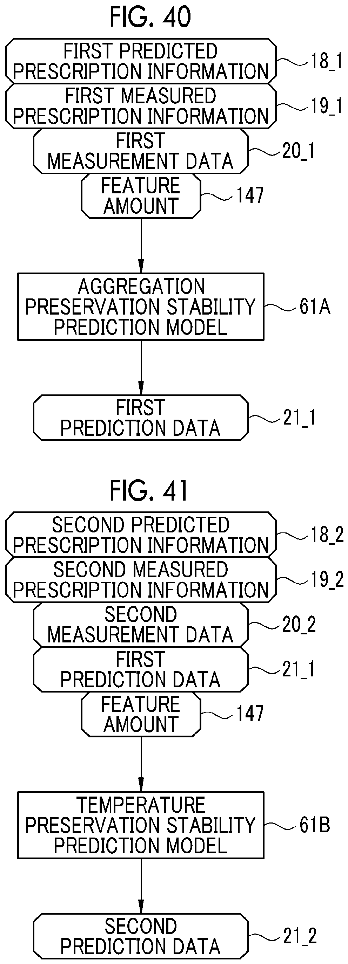

As illustrated in Figs. 40 to 42 as an example, in the third embodiment, the feature amount 147 is also input to the aggregation preservation stability prediction model 61A, the temperature preservation stability prediction model 61B, and the temporal deterioration preservation stability prediction model 61C.

As described above, in the third embodiment, the feature amount 147 derived on the basis of the antibody information 146 related to the antibody 37 included in the biopharmaceutical is also input to the preservation stability prediction model 61. Therefore, it is possible to further improve the prediction accuracy of the prediction data 21.

The feature amount 147 includes the SASA 150, the SAP 151, the SCM 152, and the PIC 153. Therefore, the prediction data 21 that more accurately indicates the preservation stability of the target solution can be output from the preservation stability prediction model 61. Further, the feature amount 147 may include at least one of the SASA 150, the SAP 151, the SCM 152, or the PIC 153.

Figs. 40 to 42 illustrate an example in which the feature amount 147 is input to the aggregation preservation stability prediction model 61A, the temperature preservation stability prediction model 61B, and the temporal deterioration preservation stability prediction model 61C according to the first embodiment. However, the present disclosure is not limited to this example. The feature amount 147 may be input to the aggregation preservation stability prediction model 130A, the temperature preservation stability prediction model 130B, and the temporal deterioration preservation stability prediction model 130C according to the second embodiment.

Instead of or in addition to the feature amount 147, the antibody information 146 may be digitized and input to the preservation stability prediction model 61 or 130.

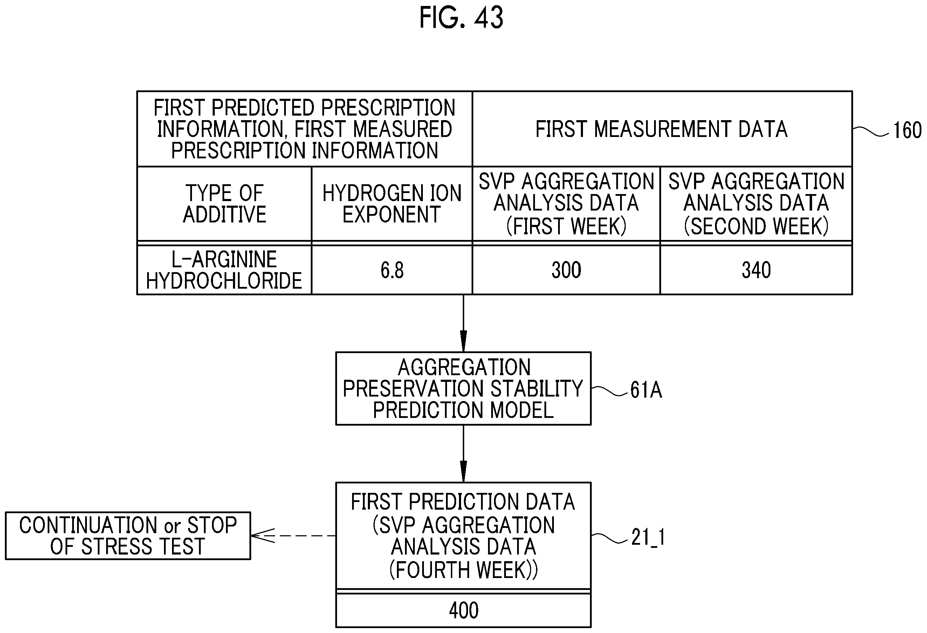

In each of the above-described embodiments, the information related to the prescription of the target solution that has not been actually adjusted is input as the predicted prescription information 18 to the preservation stability prediction model 61 or the like. However, the present disclosure is not limited thereto. As illustrated in a table 160 of Fig. 43 as an example, information related to the prescription of the prepared solution 35 that has been actually prepared and subjected to the stress test, that is, the measured prescription information 19 may be input as the predicted prescription information 18 to the preservation stability prediction model 61 or the like. In this case, the measurement data 20 is obtained by actually measuring the preservation stability of the prepared solution 35 whose measured prescription information 19 has been input as the predicted prescription information 18. Therefore, the operator determines whether to continue the stress test on the prepared solution 35 without any change or to give up and stop the stress test, on the basis of the prediction data 21 output from the preservation stability prediction model 61 or the like.

Fig. 43 illustrates a case where the first measured prescription information 19_1 as the first predicted prescription information 18_1 and the first measurement data 20_1 are input to the aggregation preservation stability prediction model 61A such that the first prediction data 21_1 is output from the aggregation preservation stability prediction model 61A. As described above, in the technology of the present disclosure, the predicted prescription information 18 and the measured prescription information 19 may be information related to the prescription of the prepared solution 35 that has been actually prepared. Therefore, the technology of the present disclosure can also be used for determining whether or not to continue the stress test on the prepared solution 35 that has been actually prepared.

In each of the above-described embodiments, three types of preservation stability prediction models of the aggregation preservation stability prediction model, the temperature preservation stability prediction model, and the temporal deterioration preservation stability prediction model are given as an example. However, the present disclosure is not limited thereto. For example, two types of preservation stability prediction models of the aggregation preservation stability prediction model and the temperature preservation stability prediction model may be provided, or two types of preservation stability prediction models of the aggregation preservation stability prediction model and the temporal deterioration preservation stability prediction model may be provided. A preservation stability prediction model for predicting other types of preservation stability, such as the preservation stability of the antibody 37 against the additive, may be added.

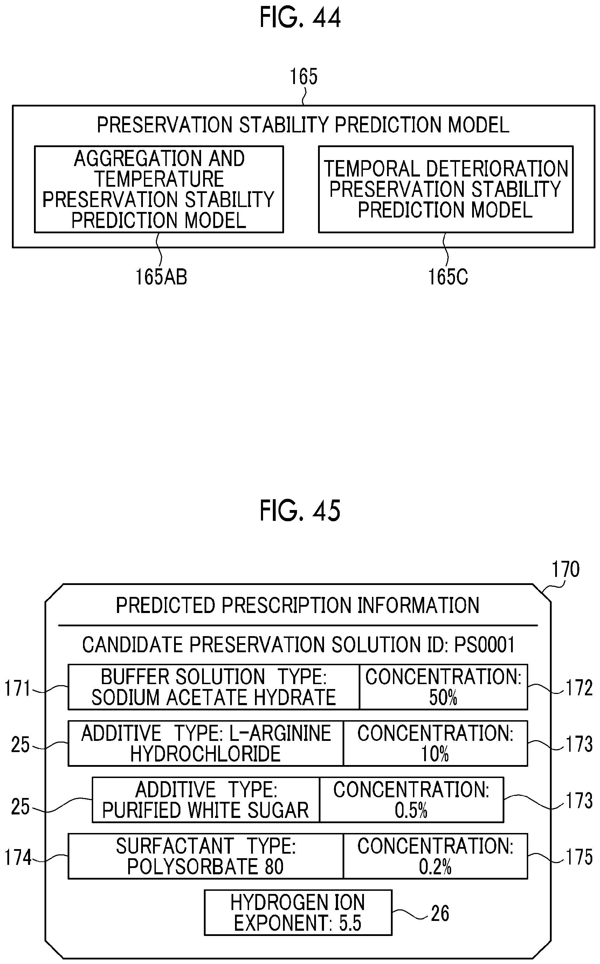

In each of the above-described embodiments, one type of preservation stability is predicted by one preservation stability prediction model. However, the present disclosure is not limited thereto. As illustrated in Fig. 44 as an example, an aggregation and temperature preservation stability prediction model 165AB that collectively predicts the preservation stability of the antibody 37 against aggregation and the preservation stability of the antibody 37 against temperature and a temporal deterioration preservation stability prediction model 165C that predicts the preservation stability of the antibody 37 against temporal deterioration may be used as the preservation stability prediction models 165. The aggregation and temperature preservation stability prediction model 165AB is a model obtained by integrating the aggregation preservation stability prediction model 61A or 130A and the temperature preservation stability prediction model 61B or 130B according to each of the above-described embodiments. The aggregation and temperature preservation stability prediction model 165AB simultaneously outputs the first prediction data 21_1 indicating the preservation stability of the antibody 37 against aggregation and the second prediction data 21_2 indicating the preservation stability of the antibody 37 against temperature.

In each of the above-described embodiments, the type 25 of the additive included in the target solution and the hydrogen ion exponent 26 of the target solution are given as examples of the predicted prescription information 18. However, the present disclosure is not limited thereto. Like predicted prescription information 170 illustrated in Fig. 45 as an example, the predicted prescription information may include a type 171 and a concentration 172 of the buffer solution included in the target solution, a concentration 173 of the additive, and a type 174 and a concentration 175 of the surfactant. The same applies to the measured prescription information 19. In some cases, a plurality of types of buffer solutions, additives, and surfactants are used as can be seen from L-arginine hydrochloride and purified white sugar which are the additives given as an example. In this case, a type and a concentration are registered for each of the plurality of types.

As described above, it is sufficient that the predicted prescription information include at least one of the types 171, 25, and 174 of the buffer solution, the additive, and the surfactant included in the candidate preservation solution, the concentrations 172, 173, and 175 of the buffer solution, the additive, and the surfactant included in the candidate preservation solution, or the hydrogen ion exponent 26 of the candidate preservation solution. Furthermore, a molecular formula of each of the buffer solution, the additive, and the surfactant may be added to the predicted prescription information.

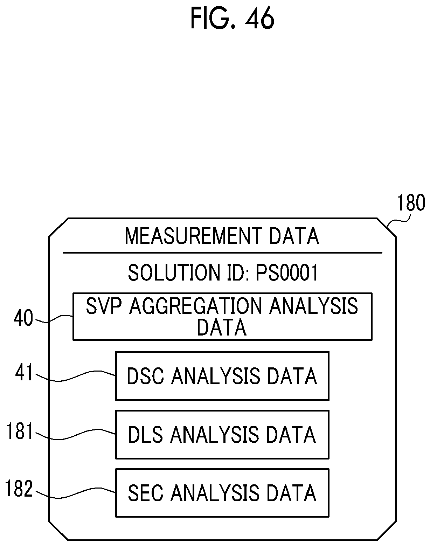

In addition, the SVP aggregation analysis data 40 and the DSC analysis data 41 are given as examples of the measurement data 20. However, the present invention is not limited thereto. Like measurement data 180 illustrated in Fig. 46 as an example, the measurement data may include analysis data (hereinafter, referred to as DLS analysis data) 181 of the antibody 37 in the prepared solution 35 by a dynamic light scattering (DLS) method and analysis data (hereinafter, referred to as SEC analysis data) 182 of the antibody 37 in the prepared solution 35 by size exclusion chromatography (SEC). The DLS analysis data 181 indicates the amount of nanoparticles of the antibody 37 in the prepared solution 35, similarly to the SVP aggregation analysis data 40. The SEC analysis data 182 indicates a molecular weight distribution of the antibody 37 in the prepared solution 35. The DLS analysis data 181 and the SEC analysis data 182 are used as measurement data indicating the preservation stability of the antibody 37 against aggregation or temporal deterioration.

In addition, it is sufficient that the measurement data includes at least one of the SVP aggregation analysis data 40, the DSC analysis data 41, the DLS analysis data 181, or the SEC analysis data 182.

The protein is not limited to the antibody 37 given as an example. Examples of the protein include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, and a sugar-chain-modified antibody.

An aspect in which the prediction data 21 is provided to be viewed by the operator is not limited to the aspect in which the prediction data 21 is displayed on the display 13. A printed matter of the prediction data 21 may be provided to the operator, or an e-mail to which the prediction data 21 has been attached may be transmitted to a portable terminal of the operator.

The pharmaceutical support server 10 may be installed in each pharmaceutical facility or may be installed in a data center independent of the pharmaceutical facility. In addition, the operator terminal 11 may be configured to have some or all of the functions of each of the processing units 65 to 67 of the pharmaceutical support server 10.

The hardware configuration of the computer constituting the pharmaceutical support server 10 according to the technology of the present disclosure can be modified in various ways. For example, the pharmaceutical support server 10 may be configured by a plurality of computers separated as hardware in order to improve processing capacity and reliability. For example, the functions of the receiving unit 65 and the RW control unit 66 and the functions of the prediction unit 67 and the delivery control unit 68 are distributed to two computers. In this case, the pharmaceutical support server 10 is configured by two computers.

As described above, the hardware configuration of the computer of the pharmaceutical support server 10 can be appropriately changed according to required performances, such as processing capacity, safety, and reliability. Further, not only the hardware but also an application program, such as the operation program 60, may be duplicated or may be dispersively stored in a plurality of storages in order to ensure safety and reliability.

In each of the above-described embodiments, for example, the following various processors can be used as a hardware structure of processing units performing various processes, such as the receiving unit 65, the RW control unit 66, the prediction unit 67, the delivery control unit 68, and the feature amount derivation unit 145. The various processors include, for example, the CPU 52 which is a general-purpose processor executing software (operation program 60) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples may be combined with each other as appropriate. In addition, it goes without saying that the present disclosure is not limited to each of the above-described embodiments, and various configurations can be adopted without departing from the gist of the present disclosure. Furthermore, the technology of the present disclosure extends to a storage medium that non-temporarily stores a program, in addition to the program.

The above descriptions and illustrations are detailed descriptions of portions related to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the specification, "A and/or B" is synonymous with "at least one of A or B". That is, "A and/or B" means only A, only B, or a combination of A and B. Further, in the specification, the same concept as "A and/or B" is applied to a case where the connection of three or more matters is expressed by "and/or".

All of the publications, the patent applications, and the technical standards described in the specification are incorporated by reference herein to the same extent as each individual document, each patent application, and each technical standard are specifically and individually stated to be incorporated by reference.

## Claims

1. A pharmaceutical support device comprising:
a processor,
wherein the processor is configured to:
use a plurality of machine learning models that output prediction data indicating preservation stability of a candidate preservation solution, which is a candidate for a preservation solution for a biopharmaceutical, at a future time point and that are provided for a plurality of types of the preservation stability, respectively;
perform a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and
input the prediction data obtained in the prediction process in a previous stage to the machine learning model in the prediction process in a subsequent stage.

2. The pharmaceutical support device according to claim 1,
wherein the plurality of machine learning models provided for the plurality of types of preservation stability, respectively, are models corresponding to at least two of preservation stability of a protein included in the biopharmaceutical against aggregation, preservation stability of the protein against temperature, and preservation stability of the protein against temporal deterioration.

3. The pharmaceutical support device according to claim 1 or 2,
wherein the processor is configured to:
input prescription information of the candidate preservation solution actually prepared to the machine learning model.

4. The pharmaceutical support device according to any one of claims 1 to 3,
wherein the machine learning model outputs a reliability degree of the prediction data together with the prediction data, and
the processor is configured to:
input the reliability degree obtained in the prediction process in the previous stage to the machine learning model in the prediction process in the subsequent stage.

5. The pharmaceutical support device according to any one of claims 1 to 4,
wherein the processor is configured to:
input a feature amount derived on the basis of protein information related to the protein included in the biopharmaceutical to the machine learning model.

6. The pharmaceutical support device according to claim 5,
wherein the feature amount includes at least one of a solvent accessible surface area of the protein, a spatial aggregation propensity of the protein, a space charge map of the protein, or an indicator showing compatibility between the protein and an additive included in the candidate preservation solution.

7. The pharmaceutical support device according to any one of claims 1 to 6,
wherein the measurement data is time-series data measured at at least two time points.

8. The pharmaceutical support device according to any one of claims 1 to 7,
wherein the prescription information related to the prescription of the candidate preservation solution to be predicted includes at least one of a type of each of a buffer solution, an additive, and a surfactant included in the candidate preservation solution, a concentration of each of the buffer solution, the additive, and the surfactant, or a hydrogen ion exponent of the candidate preservation solution.

9. The pharmaceutical support device according to any one of claims 1 to 8,
wherein the measurement data includes at least one of aggregation analysis data of sub-visible particles of the protein in the candidate preservation solution included in the biopharmaceutical, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

10. The pharmaceutical support device according to any one of claims 1 to 9,
wherein the protein included in the biopharmaceutical is an antibody.

11. A method for operating a pharmaceutical support device, the method comprising:
using a plurality of machine learning models that output prediction data indicating preservation stability of a candidate preservation solution, which is a candidate for a preservation solution for a biopharmaceutical, at a future time point and that are provided for a plurality of types of the preservation stability, respectively;
performing a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and
inputting the prediction data obtained in the prediction process in a previous stage to the machine learning model in the prediction process in a subsequent stage.

12. A program for operating a pharmaceutical support device, the program causing a computer to execute a process comprising:
using a plurality of machine learning models that output prediction data indicating preservation stability of a candidate preservation solution, which is a candidate for a preservation solution for a biopharmaceutical, at a future time point and that are provided for a plurality of types of the preservation stability, respectively;
performing a prediction process of inputting prescription information related to a prescription of a candidate preservation solution to be predicted and measurement data obtained by actually measuring the preservation stability of a candidate preservation solution actually prepared to the machine learning model such that the prediction data is output from the machine learning model in stages using the plurality of machine learning models; and
inputting the prediction data obtained in the prediction process in a previous stage to the machine learning model in the prediction process in a subsequent stage.
